# EUROPEAN PATENT APPLICATION

(11) **EP 4 495 940 A1**
(43) Date of publication of application: **22.01.2025**
(21) Application number: 24188728.0
(22) Date of filing: 15.07.2024
(51) Int. Cl.: G16H 20/70, G16H 50/20

(54) **LARGE LANGUAGE MODEL BASED CONVERSATION ENGINE FOR TREATING SYMPTOMS OF A MENTAL HEALTH DISORDER**

(30) Priority: 13.07.2023 US 202363526667 P; 14.06.2024 US 202463660437 P
(71) Applicant: Woebot Labs, Inc., San Francisco, CA 94105 (US)
(72) Inventor: DARCY, Alison, Dublin (IE); GALLAGHER, Joe, Westport (IE); CAMPELLONE, Tim, Oakland, CA (US); SACKETT, Casey, Corte Madera, CA (US); DANIELS, Jade, San Francisco, CA (US); SRINIVASAN, Sharanya, Seattle, WA (US); HARPER, Devin, Lakewood, CO (US); PAVEZ, Aaron, Arvada, CO (US); MORALES, Michelle, Franklin Square, NY (US); ODDY, Catherine, London (GB)
(74) Representative: Fish & Richardson P.C.

(57) **Abstract**

Methods, systems, and computer programs are described for using a using an LLM-based conversation engine to treat a mental health disorder. The method includes obtaining data corresponding to a communication from a user device, determining a current user context based on the obtained data, determining based on the determined user context, whether to invoke the LLM, and based on a determination to invoke the LLM: determining a prompt that is to be provided to the LLM based on the determined user context, providing input data to the LLM, the input data comprising (i) the obtained data and (ii) the determined prompt, obtaining output data, generated by the LLM based on the LLM processing the provided input data, indicating a classification of the provided input data, and using the obtained output data to determine a therapeutic treatment for the user.

## Description

### CROSS-REFERENCE TO RELATED APPLICATION

This application claims the benefit of U.S. Provisional Patent Application No. 63/526,667 titled "Large Language Model For Treating Symptoms Of A Mental Health Disorder" and U.S. Provisional Patent Application No. 63/660,437 titled "Large Language Model For Classifying Problems Associated With A Mental Health Disorder," each of which is herein incorporated by reference in their entirety.

### BACKGROUND

Digital mental health interventions (DMHIs) may include interactive software applications configured to treat symptoms of anxiety and/or depression. DMHIs are beneficial because they are designed to reduce treatment access issues for those experiencing those symptoms.

### SUMMARY

According to one innovative aspect of the present disclosure, a method for using a large language model (LLM) to treat a mental health disorder is disclosed. In one aspect, the method can include actions of obtaining, by one or more computers, data corresponding to a communication from a user device, determining, by one or more computers, a current user context based on the obtained data, determining, by one or more computers, based on the determined user context, whether to invoke the LLM, based on a determination to invoke the LLM: determining, by one or more computers, a prompt that is to be provided to the LLM based on the determined user context, providing, by one or more computers, input data to the LLM, the input data comprising (i) the obtained data and (ii) the determined prompt, obtaining, by one or more computers, output data, generated by the LLM based on the LLM processing the provided input data, indicating a classification of the provided input data, and using, by one or more computers, the obtained output data to determine a therapeutic treatment for the user.

Other versions include corresponding systems, apparatus, and computer programs to perform the actions of methods defined by instructions encoded on computer readable storage devices.

These and other versions may optionally include one or more of the following features. For instance, in some implementations, the method can further include based on a determination to not invoke the LLM: processing, by one or more computers, the obtained data through a rules engine, and using, by one or more computers, output data generated by the rules engine to determine a therapeutic treatment for the user.

In some implementations, the obtained data corresponding to a communication from a user device comprise data indicating a natural language communication.

In some implementations, the obtained data corresponding to a communication from a user device comprise data indicating selection of a preprogrammed button, an emoji, an image, or a video.

In some implementations, the determined user context is data indicating a current user status related to the user's interaction with a therapeutic application.

In some implementations, the determined user context is indicative a history of user interaction with a therapeutic application.

In some implementations, the output data indicating a classification of the provided input data generated by the LLM based on the LLM processing the provided input data.

In some implementations, the LLM has been configured using rule-example pairs.

In some implementations, the rule-example pair comprises a rule that the LLM is configured to follow and an example of the rule the LLM is configured to follow.

In some implementations, the rule-example pair configures the LLM with limits in the communication the LLM can output to the user device for review by the user.

In some implementations, the determined prompt includes data indicating a request for output data, from the LLM, as to a classification of the provided input data.

According to another innovative aspect of the present disclosure, a method for classifying a problem associated with anxiety or depression is disclosed. In one aspect, the method can include actions of obtaining, by one or more computers, data corresponding to a communication from a user, providing, by one or more computers, the obtained data as an input to a large language learning model (LLM) that has been configured to classify input data corresponding to a communication from a user into a category corresponding to a problem associated with anxiety or depression, obtaining, by one or more computers, output data generated by the LLM, based on the LLM's processing of the provided data, that is indicative of a classification of a problem associated with anxiety or depression, determining, by one or more computers, a classification of a problem associated with anxiety or depression that the user is experiencing based on the obtained output data, determining, by one or more computers and based on the determined classification, a digital therapeutic treatment for the user that is configured to treat the problem associated with anxiety or depression that the user is experiencing, and providing, by the one or more computers, the digital therapeutic to a user device of the user.

Other versions include corresponding systems, apparatus, and computer programs to perform the actions of methods defined by instructions encoded on computer readable storage devices.

These and other versions may optionally include one or more of the following features. For instance, in some implementations, the LLM has been configured using rule-example pairs.

In some implementations, the rule-example pair comprises a rule that the LLM is configured to follow and an example of the rule the LLM is configured to follow.

In some implementations, the rule-example pair configures the LLM with limits in the communication the LLM can output to the user device for review by the user.

In some implementations, the problem associated with anxiety or depression comprises at least one of a relationship problem, procrastination, grief, sleep, financial, a major sickness, a minor sickness, loneliness, addiction, pain, anxiety, depression, anger, guilt, regret, or corona.

In some implementations, determining, by one or more computers, a classification of a problem associated with anxiety or depression that the user is experiencing based on the obtained output data can include mapping, using one or more computers, the output generated by the LLM to a classification that indicates a classification of a problem associated with anxiety or depression.

In some implementations, the mapping is performed using a rules engine.

In some implementations, determining, by one or more computers, a classification of a problem associated with anxiety or depression that the user is experiencing based on the obtained output data can include evaluating, using one or more computers, natural language output generated by the LLM, and based on an evaluation of the natural language output generated by the LLM, determining that the user is in need of emotional support.

In some implementations, the method can further include obtaining, by one or more computers, second data indicating a current context associated with the user, and determining, by one or more computers, a prompt that is to be provided to the LLM based on the determined user context. In such implementations, the operation of providing, by one or more computers, the obtained data as an input to a large language learning model (LLM) that has been configured to classify input data corresponding to a communication from a user into a category corresponding to a problem associated with anxiety or depression can further include providing, by one or more computers, the obtained data, the obtained second data, and the obtained prompt as an input to a large language learning model (LLM) that has been configured to classify input data corresponding to a communication from a user, a current user context, and a prompt into a category corresponding to a problem associated with anxiety or depression.

In some implementations, the determined prompt includes data indicating a request for output data, from the LLM, as to a classification of a problem associated with anxiety or depression.

According to another innovative aspect of the present disclosure, a method for determining whether a user is in need of an empathy communication is disclosed. In one aspect, the method can include actions of obtaining, by one or more computers, first data corresponding to a communication from a user, determining, by one or more computers, second data corresponding to a prompt for the LLM based on a current user context associated with the user, providing, by one or more computers, the obtained first data and the obtained second data as inputs to a large language learning model (LLM) that has been configured to generate output data indicating whether a user is in need of an empathy communication based on processing data corresponding to (i) a communication from a user and (ii) a prompt, obtaining, by one or more computers, output data generated by the LLM, based on the LLM's processing of the obtained first data and the obtained second data, that is indicative of a whether the user is in need of an empathy communication, determining, by one or more computers and based on the obtained output data, whether the user is in need of an empathy communication, based on determining, by one or more computers, that the user is in need of an empathy communication, generating, by one or more computers, an empathy communication, and providing, by one or more computers, the empathy communication to a user device of the user.

Other versions include corresponding systems, apparatus, and computer programs to perform the actions of methods defined by instructions encoded on computer readable storage devices.

These and other versions may optionally include one or more of the following features. For instance, in some implementations, the LLM has been configured using rule-example pairs.

In some implementations, the rule-example pair comprises a rule that the LLM is configured to follow and an example of the rule the LLM is configured to follow.

In some implementations, the rule-example pair configures the LLM with limits in the communication the LLM can output to the user device for review by the user.

In some implementations, the empathy communication is a communication indicating a communication to the user of the user device that acknowledges the user's emotional state related to anxiety or depression and offers emotional support.

In some implementations, determining, by one or more computers, whether the user is in need of emotional support can include mapping, using one or more computers, the output generated by the LLM to a classification that indicates whether the user is in need of emotional support.

In some implementations, the mapping is performed using a rules engine.

In some implementations, determining, by one or more computers, whether the user is in need of emotional support can include evaluating, using one or more computers, natural language output generated by the LLM, and based on an evaluation of the natural language output generated by the LLM, determining that the user is in need of emotional support.

According to another innovative aspect of the present disclosure, a method providing an empathy communication to a user is disclosed. In one aspect, the method can include actions of obtaining, by one or more computers, first data corresponding to a communication from a user, determining, by one or more computers, second data corresponding to a prompt for the LLM based on a current user context associated with the user, providing, by one or more computers, (i) the first data and (ii) the second data as an input to a large language learning model (LLM) that has been configured to classify input data into an LLM-specific problem corresponding to a communication from a user into a category corresponding to a problem associated with anxiety or depression, obtaining, by one or more computers, output data generated by the LLM, based on the LLM's processing of the provided data, that is indicative of an LLM-specific classification of a problem associated with anxiety or depression, generating, by one or more computers, an empathy communication based on the obtained output data generated by the LLM corresponding to an LLM-specific classification, and providing, by one or more computers, the generated empathy communication to a user device of the user.

Other versions include corresponding systems, apparatus, and computer programs to perform the actions of methods defined by instructions encoded on computer readable storage devices.

These and other versions may optionally include one or more of the following features. For instance, in some implementations, the LLM-specific classification of a problem associated with anxiety or depression is a natural language description of a problem associated with anxiety or depression that the user is experiencing.

In some implementations, the method can further include mapping, using one or more computers, the LLM-specific classification that indicates a classification of a problem associated with anxiety or depression to one particular problem classification of a plurality of different problem classifications, wherein each problem classification of the plurality of different problem classifications corresponds to a predetermined problem associated with anxiety or depression.

In some implementations, the plurality of different predetermined problem classifications associated with anxiety or depression comprises at least one of a relationship problem, procrastination, grief, sleep, financial, a major sickness, a minor sickness, loneliness, addiction, pain, anxiety, depression, anger, guilt, regret, or corona.

In some implementations, the method further can further includes determining, by one or more computers, a therapeutic treatment for the user based on the particular problem classification.

In some implementations, the LLM has been configured using rule-example pairs.

In some implementations, the rule-example pair comprises a rule that the LLM is configured to follow and an example of the rule the LLM is configured to follow.

In some implementations, the rule-example pair configures the LLM with limits in the communication the LLM can output to the user device for review by the user.

According to another innovative aspect of the present disclosure, a method for generating a digital therapeutic treatment for a user that challenges one or more thoughts of a user is disclosed. In one aspect, the method can include actions of obtaining, by one or more computers, first data corresponding to a communication from a user, determining, by one or more computers, second data corresponding to a prompt for the LLM based on a current user context associated with the user, providing, by one or more computers, the obtained first data and the obtained second data as inputs to a large language learning model (LLM) that has been configured to generate output data indicating a classification of a user thought associated with a communication into a particular cognitive distortion classification of multiple cognitive distortion classifications based on processing data corresponding to (i) a communication from a user and (ii) a prompt, obtaining, by one or more computers, output data generated by the LLM, based on the LLM's processing of the obtained first data and the obtained second data, that is indicative of a classification of a user thought associated with a communication into a cognitive distortion classification, determining, by one or more computers and based on the obtained output data, a cognitive distortion classification of a user thought associated with a communication, determining, by one or more computer, a digital therapeutic treatment based on the determined cognitive distortion classification, and providing, by one or more computers, the determined digital therapeutic treatment to a user device.

Other versions include corresponding systems, apparatus, and computer programs to perform the actions of methods defined by instructions encoded on computer readable storage devices.

These and other versions may optionally include one or more of the following features. For instance, in some implementations, the multiple cognitive distortion classifications comprise emotional reasoning, labeling, mental filter, overgeneralization, blame, discounting the positive, fortune telling, all or nothing thinking, should statements, and magnification.

In some implementations, the multiple cognitive distortion classifications comprise at least one of emotional reasoning, labeling, mental filter, overgeneralization, blame, discounting the positive, fortune telling, all or nothing thinking, should statements, or magnification.

In some implementations, the determined cognitive classification comprises at least one of emotional reasoning, labeling, mental filter, overgeneralization, blame, discounting the positive, fortune telling, all or nothing thinking, should statements, or magnification.

In some implementations, a therapeutic application of a user device is configured to administer the determined digital therapeutic treatment to: receive the provided the provided digital therapeutic that was determined based on the determined cognitive distortion classification, and a administer the determined digital therapeutic treatment to the user of the user device.

According to another innovative aspect of the present disclosure, a method for keeping a user on track with a digital therapeutic treatment is disclosed. In one aspect, the method can include actions of obtaining, by one or more computers, first data corresponding to a a communication from a conversation engine, obtaining, by one or more computers, second data corresponding to a communication received from the user that is a response to the communication from the conversation engine, providing, by one or more computers, the obtained first data and the obtained second data as inputs to a large language learning model (LLM) that has been configured to generate output data indicating whether a user has strayed off track from a conversation with a conversation engine of a digital therapeutic application based on processing data corresponding to (i) a communication from a conversation engine and (ii) a communication received from the user that is a response to the communication from the conversation engine, obtaining, by one or more computers, output data generated by the LLM, based on the LLM's processing of the obtained first data and the obtained second data, that is indicative of whether a user has strayed off track from a conversation with a conversation engine of a digital therapeutic application, determining, by one or more computers and based on the obtained output data, whether the user has strayed off track from a conversation with a conversation engine of a digital therapeutic application, based on determining, by one or more computers, that the user has strayed off track from a conversation with a conversation engine of a digital therapeutic, generating, by one or more computers, a communication that steers the user back on track with the conversation with the conversation engine of the digital therapeutic, and providing, by one or more computers, the generated communication to a user device of the user.

Other versions include corresponding systems, apparatus, and computer programs to perform the actions of methods defined by instructions encoded on computer readable storage devices.

These and other versions may optionally include one or more of the following features. For instance, in some implementations, the LLM has been configured using rule-example pairs.

In some implementations, the rule-example pair comprises a rule that the LLM is configured to follow and an example of the rule the LLM is configured to follow.

In some implementations, the rule-example pair configures the LLM with limits in the communication the LLM can output to the user device for review by the user.In some implementations, the method can further include determining, by one or more computers, a prompt that is to be provided to the LLM based on the determined user context; and wherein providing, by one or more computers, the obtained first data and the obtained second data as inputs to a large language learning model (LLM) that has been configured to generate output data indicating whether a user has strayed off track from a conversation with a conversation engine of a digital therapeutic application based on processing data corresponding to (i) a first communication from a conversation engine and (ii) a communication received from the user that is a response to the communication from the conversation engine further comprises: providing, by one or more computers, the obtained first data, the obtained second data, and the determined prompt as inputs to a large language learning model (LLM) that has been configured to generate output data indicating whether a user has strayed off track from a conversation with a conversation engine of a digital therapeutic application based on processing data corresponding to (i) a first communication from a conversation engine, (ii) a communication received from the user that is a response to the communication from the conversation engine, and (iii) the determined prompt.

In some implementations, the method can further include determining, by one or more computers, a prompt that is to be provided to the LLM based on the determined user context; and wherein providing, by one or more computers, the obtained first data and the obtained second data as inputs to a large language learning model (LLM) that has been configured to generate output data indicating whether a user has strayed off track from a conversation with a conversation engine of a digital therapeutic application based on processing data corresponding to (i) a first communication from a conversation engine and (ii) a communication received from the user that is a response to the communication from the conversation engine further comprises: providing, by one or more computers, the obtained first data, the obtained second data, the determined prompt, and the current user context as inputs to a large language learning model (LLM) that has been configured to generate output data indicating whether a user has strayed off track from a conversation with a conversation engine of a digital therapeutic application based on processing data corresponding to (i) a first communication from a conversation engine, (ii) a communication received from the user that is a response to the communication from the conversation engine, (iii) the determined prompt, and (iv) a current user context. According to another innovative aspect of the present disclosure, a method for administering a digital therapeutic to a user is disclosed. In one aspect, the method can include actions of obtaining, by the user device and from the therapeutic system, therapeutic content, which was selected based on a large language learning model's classification of data corresponding to a user response into a particular problem classification of multiple problem classifications, that when rendered, by the user device and consumed by a user, treats one or more symptoms of anxiety or depression exhibited by the user, and administering, by the user device, the obtained therapeutic content as a treatment to the user, wherein administering the obtained therapeutic content as a treatment to the user comprises rendering, by the user device, the obtained digital content on a graphical user interface when the user is viewing the graphical user interface, a predetermined number of times a week for a predetermined duration.

Other versions include corresponding systems, apparatus, and computer programs to perform the actions of methods defined by instructions encoded on computer readable storage devices.

These and other versions may optionally include one or more of the following features. For instance, in some implementations, the problem classifications comprise at least one of a relationship problem, procrastination, grief, sleep, financial, a major sickness, a minor sickness, loneliness, addiction, pain, anxiety, depression, anger, guilt, regret, or corona.

In some implementations, the large language model (LLM) has been configured using rule-example pairs.

In some implementations, the rule-example pair comprises a rule that the LLM is configured to follow and an example of the rule the LLM is configured to follow.

In some implementations, the rule-example pair configures the LLM with limits in the communication the LLM can output to the user device for review by the user.

In some implementations, the obtained therapeutic content comprises one or more interpersonal psychotherapy (IPT) tools or one or more cognitive behavioral therapy (CBT) tools.

In some implementations, the obtained therapeutic content comprises one or more interpersonal psychotherapy (IPT) tools and one or more cognitive behavioral therapy (CBT) tools.

In some implementations, administering, by the user device, the obtained therapeutic content as a treatment to the user can include rendering, by the user device, the one or more interpersonal psychotherapy (IPT) tools and one or more cognitive behavioral therapy (CBT) tools in the graphical user interface of the user device a predetermined number of times a week for a duration of two to eight weeks.

In some implementations, the obtained therapeutic content comprises one or more interactive cognitive behavioral therapy (CBT) tools. In such implementations, administering, by the user device, the obtained therapeutic content as a treatment to the user can include using the user device to deliver the one or more interactive cognitive behavioral therapy (CBT) tools that comprises a natural language interaction with the user. In some implementations, the natural language interaction with the user is between a user and the large language model. In some implementations, the natural language interaction comprises (i) one or more natural language communications from the large language model to the user of the user device and (ii) one or more natural language responses from the user of the user device to the large language model.

In some implementations, the natural language interaction with the user is between the user and a conversational engine that is different from the large language model. In some implementations, the natural language interaction comprises (i) one or more natural language communications from the conversational engine to the user of the user device and (ii) one or more natural language responses from the user of the user device to the conversational engine.

In some implementations, the predetermined duration is two to eight weeks.

In some implementations, the predetermined duration is at least 8 weeks.

In some implementations, the therapeutic content is administered for at least 5 minutes during each of the predetermined number of times per week.

In some implementations, the obtained therapeutic content is administered at least 10 minutes during each of the predetermined number of times per week.

In some implementations, the predetermined number of times a week for a predetermined duration comprises at least 4 weeks of an 8-week time period.

These and other innovative aspects of the present disclosure are readily apparent in view of the detailed description, the accompanying drawings, and the claims.

### BRIEF DESCRIPTION OF THE DRAWINGS

FIG. 1 is a diagram of an example of a system for generating, dosing, and administering a digital therapeutic to treat one or more symptoms related to anxiety or depression.
FIG. 2 is a flowchart of an example of a process that can be executed by a diagnosis engine, in accordance with one aspect of the present disclosure.
FIG. 3 is a flowchart of an example of a process that can be executed by a dosing engine, in accordance with one aspect of the present disclosure.
FIG. 4 is a flowchart of an example of a process that can be executed by a treatment engine, in accordance with one aspect of the present disclosure.
FIG. 5 is a flowchart of an example of a process that can executed by a persistent symptom mitigation engine, in accordance with one aspect of the present disclosure.
FIG. 6 is a diagram of an example of a user device that is configured to generate, dose, and administer a digital therapeutic to treat one or more symptoms related to anxiety or depression.
FIG. 7 is a flowchart of a process that can be executed by an LLM-based conversation engine that comprise a rules-based conversation agent that can selectively interact with a large language model (LLM) to increase the effectiveness of a digital therapeutic treatment, in accordance with one aspect of the present disclosure.
FIG. 8 is a flowchart of a process for determining whether to invoke a large language model (LLM) to treat a mental health disorder, in accordance with one aspect of the present disclosure.
FIG. 9 is a flowchart of a process for using an LLM to classify a problem a user is experiencing related to anxiety or depression, in accordance with one aspect of the present disclosure.
FIG. 10 is a flowchart of a process for providing empathy to a user based on classification of a user communication by an LLM, in accordance with one aspect of the present disclosure.
FIG. 11 is a flowchart of another process for providing empathy to a user based on a classification of a user communication into a problem associated with anxiety or depression, in accordance with one aspect of the present disclosure.
FIG. 12 is a flowchart of a process for generating a thought challenging treatment for a user, in accordance with one aspect of the present disclosure.
FIG. 13 is a flowchart of a process for keeping a user on track with a conversation with a conversation engine of a digital therapeutic application, in accordance with one aspect of the present disclosure.
FIG. 14 is a flowchart of a process for administering a digital therapeutic selected based on the output of a large language model to a user, in accordance with one aspect of the present disclosure.
FIG. 15 is a block diagram of system components that can be used to implement a system for generating, dosing, and administering a digital therapeutic to treat one or more symptoms related to anxiety or depression.
FIG. 16 is a CONSORT diagram describing participant selection and participation in a study that used aspects of the present disclosure to treat anxiety or depression using a rules-based conversation engine.
FIG. 17 is a CONSORT diagram describing participate selection and participation in a study that used aspects of the present disclosure to treat anxiety or depression using an LLM-based conversation engine.

### DETAILED DESCRIPTION

The present disclosure is directed towards methods, systems, and computer programs for treating one or more symptoms of anxiety or depression for patients diagnosed with depression and/or anxiety, or for users with symptoms of depression or anxiety. A symptom of anxiety or depression can include, for example, a mental or physical feature of an individual which is regarded as indicating a presence of anxiety or depression. In some implementations, the present disclosure is configured to treat one or more symptoms of anxiety or depression using particularly configured digital mental health interventions (DMHIs) that are administered to an individual during a prescribed or recommended dosing schedule. An example of a DMHI can include, for example, administering a digital therapeutic to the user a prescribed number of times during a prescribed time period. In one implementation, the prescribed dosing schedule is one or more times per week for two to eight weeks. As described herein, "prescribed" may relate to prescription by a health care provider, or recommendation by the DMHI to the user. In some implementations, a DMHI is performed using the system 100 or user device 610.

In some implementations, the DMHI can occur by using a conversational agent to optimize engagement with the user (e.g., a patient diagnosed as exhibiting one or more symptoms of anxiety or depression). The conversational agent can include, for example, a conversational engine that is configured to engage in natural language communication with the user. Engaging in natural language communication with the user can include, for example, using a natural language processor for deciphering natural language inputs received from the user, generating a natural language response to the user based on the received natural language input, and communicating the generated natural language response to the user.

Multiple benefits are achieved by use of the conversational agent, as the conversational agent is configured to communicate with the user conversationally using the conversation engine in a manner that exhibits a friendly "personality" in an effort to establish a relationship, or other alliance, with the user. The conversational agent is designed to use this relationship to cause the user to developed a therapeutic alliance with the conversational agent, engage the virtual entity in a manner that provides characterizations of, e.g., the user's mood, and then to adhere to the treatment ultimately administered to the user via the virtual entity based on, e.g., the user's mood, as deciphered by the virtual entity's natural language processing performed by the virtual entity's conversation engine, mood evaluation engine, or both. For instance, the virtual entity may utilized BERT, transformers, or other NLP models to analyze a user's natural language responses, and identify problems or issues to address.

Additional benefits can be achieved by implementation of a persistent symptom mitigation engine. The persistent symptom mitigation engine dynamically measure an individual's current mood, changes in the individual's mood, or trends in the changes of the individual's mood after treatment of one or more symptoms of anxiety or depression has begun. For example, the persistent symptom mitigation engine can evaluate feedback (for instance in a natural language response), from an individual, after the administration of treatment of a digital therapeutic. Then, based on the evaluation of this received feedback, the persistent symptom mitigation engine can determine an individual's current mood, changes in the individual's mood, or trends in the changes of the individual's mood. This dynamic measurement of the level of one or more symptoms yields clinical and technological benefits.

The clinical benefits of this dynamic measurement include the dynamic adjustment of a treatment or dosing schedule to be made based on the individual's response to an initial treatment. Such benefits enable a digital therapeutic or a dosing schedule for the digital therapeutic to be dynamically customized based on an individual's responsiveness to an initial treatment. This dynamically customized treatment and/or dosing schedule helps to improve the medical outcomes of an individual being treated using the digital therapeutic of the present disclosure, as the treatment and/or dosing schedule is tuned to the precise mental and/or emotional state of an individual.

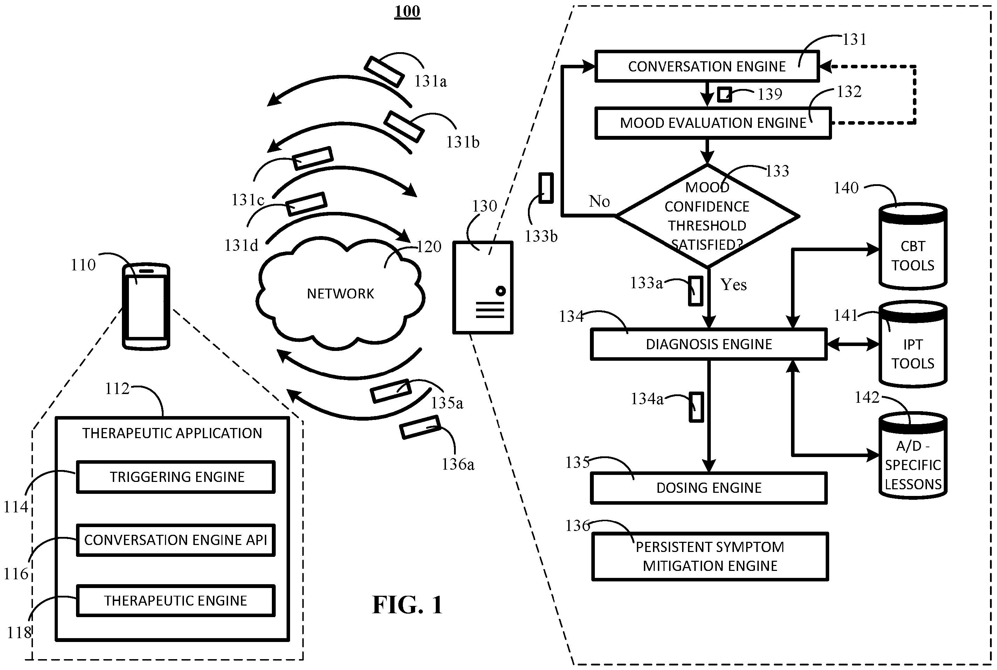

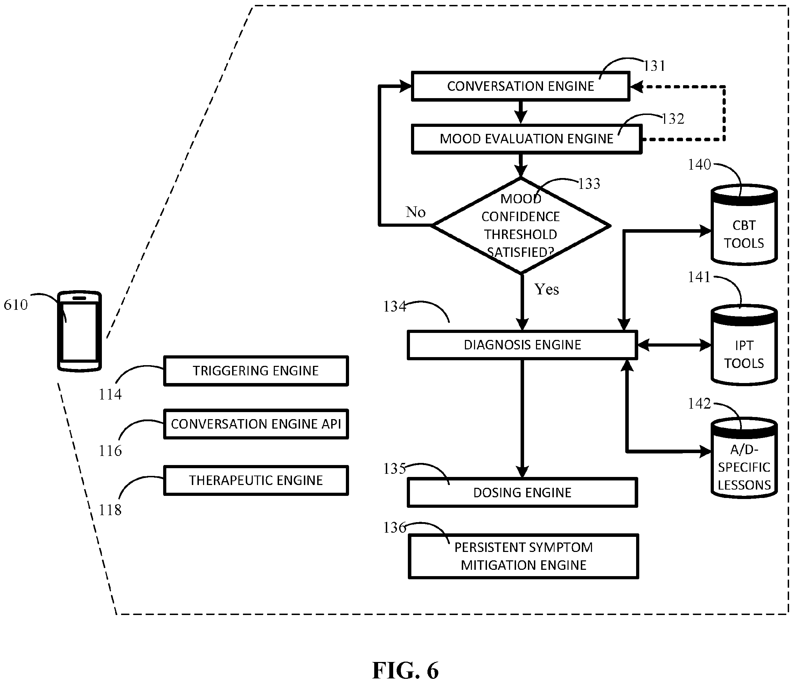

The technological benefits of this dynamic measurement includes the conservation of resources that is achieved by using the persistent symptom mitigation engine. In particular, persistent symptom mitigation engine is capable of terminating administration of a digital therapeutic treatment before the end of a prescribed therapeutic regimen if the persistent system mitigation engine determines that the user current mood, change in mood, or trend in mood change indicates that an individual's symptoms of anxiety or depression have been sufficiently mitigated. When administration of a treatment of a digital therapeutic is terminated before the end of a prescribed therapeutic regimen, the system of the present disclosure conserves the resources that would have been spent to administer the digital therapeutic for the remainder of the therapeutic regimen, thus achieving a technical improvement to the computing system. Specifically, there will be a reduction in the amount of processor (e.g., CPU, GPU, etc.) cycles executed, memory resources utilized, and bandwidth utilized when administration of the digital therapeutic is terminated earlier than a prescribed therapeutic regimen. This reduction in resources is achieved on the server side (e.g., server 130 does not cause the user device 110 to continue administering treatment at stage 440), on the user device side (e.g., user device 110 does not continue administering treatment at stage 440), or both. In some instances, where the present disclosure is fully implemented on the user device 110 such as in FIG. 6 (as opposed to the split user device side / server side architecture of FIG. 1), the user device 110 realizes a reduction in resources in both of the aforementioned scenarios.

In addition to the benefits and/or technical improvements identified above, there is a need, in the art, for the administration of such digital therapeutics. For example, an individual may be unable to take prescription medicine for depression or anxiety due to side effects, allergies, or other reasons. In such instances, methods of the present disclosure can meet this need as an alternative treatment for individuals who may not have access to therapists, and/or may be unwilling to engage in therapy with a live person.

Furthermore, aspects of the present disclosure are directed towards methods, systems, and computer programs for treating anxiety or depression using an LLM-based conversation engine. In such implementations, the LLM-based conversation engine is configured to selectively leverage the LLM along with a rules-based conversation agent in order to assist with the treatment of a user for symptoms of anxiety or depression using a digital therapeutic. In other implementations, the LLM-based conversation engine is configured to use the LLM to assist with the treatment for symptoms of anxiety or depression using a digital therapeutic without the user of a rules-based conversation agent.

The LLM-based conversation engine provides a number of advantages over conventional conversation engines that used solely rules-based conversation agents. For example, in some implementations, the LLM-based conversation engine of the present disclosure provides an improved treatment of anxiety or depression by improving the classification of a problem associated with anxiety or depression that a user is currently experiencing. In particular, aspects of the present disclosure configure a large language learning model (LLM) of the LLM-based conversation engine to obtain a communication from a user and process the obtained communication from the user through the LLM that has been trained to classify data corresponding to a user communication into a particular problem classification of multiple different problem classifications. On this point, experimental results have shown that an LLM of the LLM-based conversation engine is configured to classify data corresponding to a user communication into a particular problem classification resulted in the LLM accurately classifying a problem experienced by a user 98% of the time, whereas conventional rules-based approaches only accurately classified a problem experienced by a user 69% of the time.

Furthermore, the present disclosure is not limited to a LLM of an LLM-based conversation engine that classifies data corresponding to a user communication into a particular classification corresponding to a problem associated with anxiety or depression. Instead, the LLM of the LLM-based conversation engine of the present disclosure can also be configured to classify data corresponding to a user communication into a mood classification. For example, the LLM of the LLM-based conversation engine can be configured to process data corresponding to a user communication and classify the data corresponding to the user communication into a category of a particular mood from multiple different moods. Then, therapeutic content can be selected to treat anxiety or depression in the user based on the mood classification indicated by the output of the LLM of the LLM-based conversation engine.

In yet other implementations, the LLM of the LLM-based conversation engine is configured to classify data corresponding to a user communication into a particular classification corresponding to whether a user that submitted the communication is in need of empathy. In such instances, the LLM of the LLM-based conversation engine is configured to generate and provide an empathy communication to the user to communicate to the user that the LLM-based conversation engine has detected the user's need of empathy and that the LLM-based conversation engine or, e.g., the therapeutic application as a whole offers emotional support. Such an empathy communication can help the LLM-based conversation engine build a therapeutic relationship with the user that drives adherence and elicits better response data that can be used to more accurately diagnose and treat the user's anxiety or depression.

FIG. 1 is a diagram of an example of a system 100 for generating, dosing, and administering a digital therapeutic to treat one or more symptoms of anxiety or depression.

The system 100 can include a user device 110, a network 120, and an application server 130. The user device 110 can include a smartphone, tablet computer, a laptop computer, a desktop computer, a smart television, a smartwatch, or the like. The user device 110 can include therapeutic application 112 that is installed, thereon. The therapeutic application 112 can include a treatment triggering engine 114, a conversation engine API 116, and a therapeutic engine 118. The network 120 can include one or more of a wired or wireless local area network, a short-wave radio network, a cellular network, the Internet, or any combination thereof. The application server 130 can include one or more computers configured to store and execute the respective engines 131, 132, 133, 134, 135, 136, shown in FIG. 1. In some implementations as shown in FIG. 1, the databases 140, 141, 142 may be stored within one or more computers of the application server 130. However, in other implementations, the databases 140, 141, 142 may be hosted by one or more remote computers. In such implementations, application server 130 can be configured to interact with the databases 140, 141, 142 by communicating with the one or more remote computers. In the example of FIG. 1, the databases 140, 141, 142 include lessons that can be selected and provided to a user device 110 as part of a digital therapeutic configured to treat one or more symptoms of anxiety or depression.

With reference to FIG. 1, the therapeutic application 112 is installed on the user device 110. Then, at some point in time after installation of the therapeutic application 112 on the user device 110, execution of the system 100 begins with the triggering engine 114 triggering a therapeutic treatment session. A therapeutic treatment session can be described as the time period that begins with the therapeutic application 112 initiating interaction with an individual (e.g., user of user device 110) through either (a) completion of a therapeutic treatment or (b) a determination, by the therapeutic application, that the individual does not need a therapeutic treatment. For purposes of this disclosure, "individual" is understood to be the "user" of the user device 110 that is being receiving treatment for one or more symptoms of a anxiety or depression from the therapeutic application. Accordingly, the words "individual" and "user" are intended to have the same meaning and may be used interchangeably.

The triggering engine 114 is configured to drive adherence of a user to a therapeutic treatment for one or more symptoms of anxiety or depression. The trigger engine 114 achieves this by autonomously initiating interactions with the user of the user device 110 to determine an emotional state of the user. In some implementations, initiating interaction with the user can include, for example, receiving notifications 131a (e.g., push notifications) and generating alerts for output by the user device 110. Such alerts can, for example, encouraging a user to check-in with the therapeutic application 112. Check-in can include, for example, a user opening up the therapeutic application 112 and interacting with the therapeutic application 112.

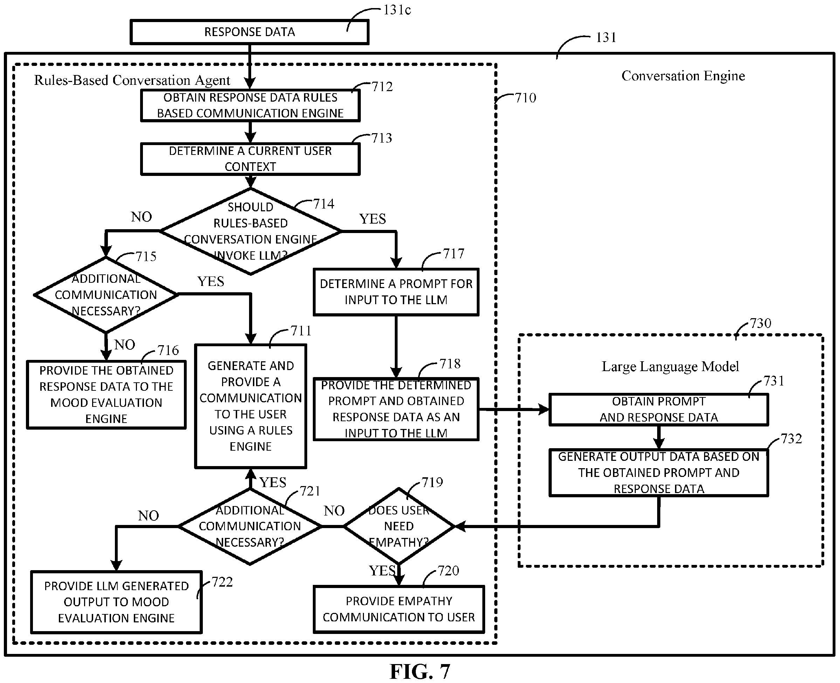

In accordance with one aspect of the present disclosure, interactions with the the therapeutic application 112 can be described with reference to the flowchart of FIG. 7, which is a flowchart that describes a process that can be executed by a conversation engine that comprise a rules-based conversation agent that can selectively interact with a large language model (LLM) to increase the effectiveness of a digital therapeutic treatment. In some implementations, interactions with the therapeutic application 112 can begin with the conversation engine 131 communicating 131b with the user of the user device 110 via the conversation engine API 116. For example, the conversation engine 131 can cause a rules-based conversation agent 710 to generate and provide a communication to the user using a rules engine 711. Generating a communication using a rules engine can include, for example, the rules-based conversation agent determining a current user context then selecting a predetermined communication based on a set of predetermined rules applied to the current user context. A current user context can include, for example, a user's status related to the user's interaction with the therapeutic application 112. For example, the rules-based conversation agent may determine that the current user context is that it is (i) morning time and the (ii) the user has not interacted with the conversation therapeutic application 112 for more than threshold period of time (e.g., 48 hours). In such instances, the rules-based conversation engine 710 can generate a communication to the user by applying a set of rules to the aforementioned user context to generate a general inquiry such as, e.g., "Good morning, I have not heard from you for a few days. How are you doing today?" Such a communication may be transmitted to the user as a communication 131b and operate as, e.g., a general inquiry, by the conversation engine 131, about the user's mood. In some implementations, the communication 131b is transmitted by the server 130 across the network 120 to the user device 110.

The user device 110 can obtain the communication 131b and generate output data that presents the communication 131b to the user of the user device 110. The output data can be presented to the user of the user device 110 in a number of different ways. For example, the output data can include displaying a message, from the conversation engine 131, to the user in an interface in a messaging application format that shows dialogue from the conversation engine 131 on one side of the messaging application interface and dialogue from the user, when received, on the other side of the messaging application interface. However, the present disclosure need not be so limited and, instead, in some implementations, the communications 131a can be presented to the user via audio output from a speaker. In some implementations, for example, the conversation engine 131 can transmit a communication 131b that asks the user "Good morning, I have not heard from you for a few days. How are you doing today?" The user of the user device 110 can input a response 131c to the communication 131b into the therapeutic application 112. The conversation engine API 116 can transmit the response 131c to the server 130 via the network 120.

The rules-based conversation agent 710 of the conversation engine 131 can obtain the response data 131c (712). The rules-based conversation agent 710 can also determine a current user context 713. The current user context can include, for example, a current state associated with the user of the user device 110 such as, e.g., time of day, period of time since the user last interacted with the therapeutic application 112, frequency of user interaction with the therapeutic application 112, or the like. Then, based on the response data 131c obtained at stage 712 and the user context determined at stage 713, the rules-based conversation agent 710 can determine whether to invoke an LLM 730 at stage 714.

If the rules-based conversation agent 710 determines to not invoke the LLM 730, the rules-based conversation can then determine whether additional communication with the user of the user device 110 is necessary at stage 715. If the rules-based conversation agent 710 determines that additional communication with the user of the user device 110 is necessary at decisioning engine 715, then the rules-based communication agent 710 can generate and provide a communication to the user using a rules engine at stage 711. For example, in some implementations, the rules-based conversation agent 710 may determine that additional communication with the user of the user device 710 is necessary if an evaluation of the response data 712, by the rules-based conversation agent 710, does not satisfy a threshold level of clarity. For example, the rules-based conversation agent 710 can use natural language process techniques to analyze the obtained response data 712. And, in some instances, the rules-based conversation agent 710 can determine that the obtained response data is indecipherable. Response data may be indecipherable if, e.g., the received response is misspelled, grammatically incorrect, a combination of one or more seemingly random letters, symbols, words, emojis, or the like. In such instances, the rules-based conversation agent 710 can generate and provide another communication to the user using the rules engine 711. Alternatively, if the rules-based conversation agent determines that additional communication with the user of the user device 110 is not necessary at stage 715, then the rules-based conversation agent 710 can provide the obtained response data 131c to the mood evaluation engine 132 at 716.

In some instances, however, the rules-based conversation agent 710 can determine, using decisioning engine 714, to invoke an LLM 730. In some implementations, the rules-based conversation agent 710 can determine to invoke an LLM 730 based on the current user context determined at stage 713. For example, the rules-based conversation agent 710 can process the current user context 713 through a rules-engine that is configured to map the current context into one of two different classifications. These classifications can include (i) a first classification indicating that an LLM 730 should not be invoked or (ii) a second classification indicating that an LLM 730 is to be invoked. Accordingly, in such implementations, if the rules-based conversation agent 710 maps the current user context to a classification indicating that the LLM 730 is to be invoked, then the rules-based conversation agent 710 can determine to invoke the LLM 730 at 714.

Upon a determination to invoke the LLM 730, the rules-based conversation agent 710 can determine a prompt for input to the LLM 730. A prompt for an LLM is data that provides an indication to an LLM as to what type of output data is sought from the LLM. In some implementations, prompt may be one or more words, an instruction, a question, or an other type of data that provides an indication to the LLM as to what type of output data is sought from the LLM. An example of a prompt for input to the LLM 730 may be, e.g., "Is the user experiencing symptoms of anxiety or depression?". Another example of a prompt for input to the LLM 730 may be, e.g., "Please determine a problem the user is experiencing related to anxiety or depression?" Yet another example of a prompt for input to the LLM 730 may be, e.g., "Does the user need empathy?" The present disclosure is not limited to these prompts or any finite set of prompts. However, these are examples of data that can be input to an LLM to provide an indication to the LLM as to the output data sought from the LLM.

The rules-based conversation agent 710 can determine a prompt for input to the LLM 730 based on (i) the obtained response data 131c obtained at stage 712 and (ii) the current user context determined at stage 713. In some implementations, the rules-based conversation agent 710 can use a rules engine that is configured to map each user context of a plurality of predetermined user contexts to a particular LLM prompt. For example, the rules-based conversation agent 710 may obtain response data from a user that stated "I'm feeling down today" and determine a current user context of, e.g., "no prior communication from the user for more than 24 hours" and map that response data - current user pair to an LLM prompt of "Please determine whether the user is experiencing symptoms of anxiety or depression?". Accordingly, current user context can be mapped to one or more particular prompts of a predetermined set of prompts. This means that for each response from a user, the current user context associated with the response can be mapped to one prompt or multiple prompts. In the case of multiple prompts being determined, the rules-bases conversation agent 710 can invoke the LLM once for each prompt that was selected, thus providing the potentiala for an LLM to be invoked multiple times for each response from the user.

The predetermined set of prompts can include, for example, a request to classify a particular problem the user is experiencing related to anxiety or depression, a request to determine whether a user is in need of empathy, a request to determine whether a user is strayed off track from a conversation with the conversation engine and needs to be brought back on track with the conversation, a request to classify a user thought into a particular cognitive distortion classification, or any other request for information that is sought from the LLM.

The rules-based conversation agent 710 can then provide the determined prompt (e.g., "Please determine whether the user is experiencing symptoms of anxiety or depression?") and response data obtained at stage 713 as inputs to the LLM 730 at stage 718. The LLM 730 can obtain the prompt and response data as inputs at stage 731. In some implementations, the current context may also be input to the LLM in addition to the prompt and response data.

The LLM 730 is configured to process input data corresponding to a prompt and response data and then generate, at stage 732, output data based on the LLM's 730 processing of the obtained prompt and response data. Specifically, in some implementations, the present disclosure configures the LLM 730 by training the LLM using a series of rule-example pairs. For example, the LLM can be configured using a prompt that includes one or more rules instructing the LLM to identify "relationship problems" and also includes one or more examples of relationship problems such as "mother and daughter," "father and son," "husband and wife," "girlfriend and boyfriend," "brother and sister," etc. A more pertinent example of a rule-example pair may include, for example, a rule of "user has depression" and also include one or more examples of responses previously provided from a user that has depression. Rule-example pairs configure the LLM 730 by using the examples to provide context to the rule provided to the LLM 730 and, as a result, enable the LLM 730 to contextualize the rule provided to the LLM. Such rule-example pairs can be generated, input as prompts to the LLM, and used to configure the LLM to detect and classify problems a user is experiencing related to anxiety and/or depression. Importantly, these examples of rule-example pairs are not provided as verbatim rule-example pairs that would be used to configured the LLM 730. It is not practical, for purposes of this disclosure, to fully enumerate all forms of rule-example pairs that would be used to configured the LLM 730 of the present disclosure. Instead, these examples are provided to disclose and support the scope of rule-example pairs that are used to configure the LLM 730 of the present disclosure.

The rules-based conversation agent 710 can obtain the output generated by the LLM 730 and then determine using decisioning engine 719 whether the output data generated by the LLM indicates that the user needs empathy. The rules-based conversation agent's 710 determination as to whether the user needs empathy is based on the rules-based conversation agent's 710 evaluation of the output data generated by the LLM 730 at stage 732. Like the other examples above, the rules-based conversation agent 710 can use a rules engine to map output data generated by the LLM based on processing of the prompt determined at stage 717 and the response data obtained at 712 to classifications that comprise (i) user needs empathy or (ii) user does not need empathy. If the output data generated by the LLM 730 indicates that the user needs empathy, the rules-based conversation agent 710 can generate and provide an empathy communication to the user of the user device 110 at stage 720. The empathy communication is a caring response to the emotional state of the user that is detected based on the LLM's 730 processing of the response data and current user context. This empathy communication is one or more communications that express detection of the user's anxiety or depression and indicate that the conversation engine 131 is vicariously experiencing the feelings and/or thoughts associated with the user's anxiety or depression. For example, the empathy communication be a communication that indicates an acknowledgment of the user's emotional state related to anxiety or depression and offers emotional support. This empathy communication is helpful to the user because it helps the user to feel like the user is not alone in the user's battle with the symptoms of anxiety or depression.

It is not necessary for the conversation engine 131 actually "experience" feelings and/or thoughts associated with the user's anxiety or depression. Instead, it is sufficient for the rules-based conversation agent 710 of the conversation engine 131 to generate a communication that provides an indication that the conversation engine 131 is experiencing the feelings and/or thoughts associated with the user's anxiety or depression. Accordingly, the conversation engine 131 can provide an empathy communication without being able to actually experience or feel as a human would.

If the decisioning engine 719 determines that the user needs empathy, the empathy communication is provided to the user device 110 of the user at stage 720. This empathy communication will then trigger the user to provide subsequent response data that will be received and processed via the conversation engine 131 using the process of FIG. 7 in the same manner described above with reference to response data 131c. Accordingly, this empathy communication can help the conversation engine 131 build a therapeutic relationship with the user that drives adherence and elicits better response data that can be used to more accurately diagnose and treat the user's anxiety or depression.

This is, however, only one example of how the rules-based conversation agent 710 can determine if the user is in need of an empathy communication. However, the present disclosure is not so limited. For example, in some implementations, the rules-based conversation agent 710 can determine a prompt at stage 718 that requests classification of a user communication into a problem associated with anxiety or depression. In such implementations, the prompt can be a request for an LLM-specific classification of the problem associated with anxiety or depression that a user is experiencing. In such instances, the output generated by the LLM at stage 732 can include an LLM-specific classification that is, e.g., a natural language description of a problem associated with anxiety or depression that a user is experiencing. The LLM 730 can provide this generated output data to the decisioning engine 719. Then, the decisioning engine 719 of the rules-based conversation agent 710 can determine whether the user is in need of an empathy communication. Such a determination may be based on a natural language analysis of the generated output of the LLM. In such instances, if the decisioning engine 719 determines that the user is in need of empathy, the rules-based conversation agent 710 can generate an empathy communication that includes the LLM-specific classification of the problem associated with the problem the user is experiencing. This empathy communication can include, for example, a natural language communication generated by the LLM that paraphrases the problem associated with anxiety or depression that the user is experiencing. This communication to the user can provide empathy to the user, as it shows that that therapeutic application (via the LLM-based conversation engine) understands the problems associated with anxiety or depression that the user is experiencing and conveys an implicit message to the user that the user is not alone. This can help sooth the user's symptoms of anxiety or depression and strengthen the therapeutic relationship between the user and the therapeutic application, thus improving adherence.

Alternatively, if the rules-based conversation agent 710 determines at stage 719 that the user does not need empathy, then the rules-based conversation agent 710 can determine whether additional communication with the user is necessary using the decisioning engine 721.

If the rules-based conversation agent 710 determines that additional communication with the user of the user device 110 is necessary at decisioning engine 721, then the rules-based communication agent 710 can generate and provide a communication to the user using a rules engine at stage 711. In some implementations, the rules-based conversation agent 710 may determine that additional communication with the user of the user device 710 is necessary if it is determined that output data generated the LLM 730 is not sufficient to provide to the mood evaluation engine 722. For example, in some implementations, the rules-based conversation agent 710 may process the output data generated by the LLM 730 and determine that the LLM 730 needs additional information in order generate output data that can be used to classify the user's current mood. In such instances, the rules-based conversation agent 710 can generate and provide a follow-up communication to the user at stage 711 to elicit additional response data that can be subsequently obtained by the conversation engine 131 and processed through the flowchart of FIG. 7, as described above with respect to response 131c.

In yet other implementations, the rules-based therapeutic agent 710 can determine that additional communication with the user is necessary to get the user back on track with the conversation between the user and the conversation engine 131. For example, in some implementations, each response from the user is evaluated to determine whether the user has strayed from the communication between the user and the conversation engine 131 and needs to be brought back on track. This can be achieved, e.g., by determining to invoke the LLM at stage 174 and then determining a prompt based on the current user context that indicates that a back on track evaluation needs to be performed. In such instances, a communication from the conversation engine 131 (that triggered the user's response at stage 712), the user's response to the communication from the conversation engine 131 received at stage 712, , and the determined prompt (e.g., determine if the user needs to be brought back on track) are provided as input to the LLM at stage 718. The LLM 730 can obtain the communication from the conversation engine, the user's response to the communication from the conversation engine, and the prompt, process the communication from the conversation engine, the user's response to the communication from the conversation engine, and the prompt, and then generate output data indicating whether the user has strayed from a conversation between the user and the conversation engine. This output data can be provided to the decisioning engine 721, which is configured to determine whether additional communication with the user is necessary. Here, if the rules-based conversation engine 710 executes the decisioning engine 721 and determines, based on the output data generated by the LLM, that the user has strayed from a conversation between the user and the conversation engine 131, then the rules-based conversation agent 710 can generate a communication that is configured to steer the user back on track to the conversation between the user and the conversation engine 131.

Alternatively, if the rules-based conversation engine determines at 721 that additional communication with the user of the user device 110 is not necessary based on the output data generated by the LLM 730, then the rules-based conversation agent 710 can provide the output data generated by the LLM 730 to the mood evaluation engine 722. In some implementations, the rules-based conversation agent 710 may determine that additional communication with the user of the user device 710 is not necessary if it is determined that output data generated the LLM 730 is sufficient to provide to the mood evaluation engine 722.

The rules-based decisioning agent 710 may determine, using the decisioning engine 721, that additional communication with the user is unnecessary if, e.g., the output generated by the LLM at stage 732 adequately classifies a user response into a problem associated with anxiety or depression, the output data generated by the LLM at stage 732 adequately classifies the output data generated by the LLM into a particular cognitive distortion classification, or the like. In such instances, the output data may be provided to the mood evaluation for mapping to a particular mood. Then, the mapped mood can be used by the diagnosis engine to determine therapeutic content that is to be used for the digital therapeutic.

In yet other implementations, the rules-based conversation agent 710 may be need to use a rules-engine to map output data generated by the LLM at stage 732 from an LLM-specific classification to a particular problem associated with anxiety or depression of multiple predetermined problems associated with anxiety or depression. The predetermined problems associated with anxiety or depression can include one or more of a relationship problem, procrastination, grief, sleep, financial, a major sickness, a minor sickness, loneliness, addiction, pain, anxiety, depression, anger, guilt, regret, or corona. Then, once this mapping has occurred, the conversation engine can provide the particular predetermined problem classification as an input to the mood evaluation engine for mapping to a particular mood. Then, the mapped mood can be used by the diagnosis engine to determine therapeutic content that is to be used for the digital therapeutic.

The conversation engine 131 can continue to communicate with the user in an iterative manner by using stage 711 or 720 to generate and provide a communication to the user of the user device 110 to solicit a response from the user of the user device can be processed through the flowchart of FIG. 7 until a sufficient response is generated that can be provided to the mood evaluation engine 132 for further processing. In the above example, the examples of communications between the conversation engine 131 and the user of the user device 110 included natural language communications. However, the present disclosure is not so limited. Instead, in some implementations, the communications between the conversation engine 131 and the user of the user device 110 can include other forms of communications. For example, in some implementations, the conversation engine 131 can provide a plurality of pre-programmed button options. Pre-programmed buttons can be represented in a graphical user interface of the therapeutic application 112 using a selectable graphical icon. Each pre-programmed button can correspond to an attribute of an emotional state (e.g., a mood). Emotional states that can be associated with a pre-programmed button can include, e.g., anxious, really anxious, sick, really sick, angry, really angry, happy, really happy, content, sad, really sad, depressed, really depressed, okay, tired, really tired, or the like. In such implementation, the user's response to a communication from the conversation engine 131 that includes pre-programmed buttons can include a selection of one or more of the pre-preprogrammed buttons. Likewise, in some implementations, communications from the conversation engine 131 to the user of the user device or communications from user of the user device 110 to the conversation engine can include emojis, images (e.g., GIFs, JPEGs, etc.), videos, hyperlinks, or any other form of content. If a hyperlink is provided in a communication, the hyperlink can be accessed by the conversation engine 131 and the contents of a website or other file associated with the hyperlink may be analyzed using the process described with respect to FIG. 7.

The conversation engine 131 can provide the output data generated by the LLM 730, the response data 131c, or both, as an input to the mood evaluation engine 132. For example, in the context of stage 716 where the LLM 730 was not invoked, the rules-based conversation agent 710 can provide the response data 131c to the mood evaluation engine 132. In such instances, the mood evaluation engine 132 can evaluate the response 131c and determine, based on the response 131c, a likely mood of the user of the user device 110. For example, in some implementations, the mood evaluation engine 132 can provide the response 131c as an input to a machine learning model that has been trained to process response 131c data and generate output data corresponding to a mood of the user based on processing of the response 131c data. Data corresponding to the mood of the user device 110 can include, for example, data indicating a likely mood of the user and a confidence score indicating a confidence of the machine learning model that the predicted mood is correct. In such implementations, the machine learning model can be trained on training data items corresponding to response data provided by prior patients, response data provided by patients of a clinical trial, or the like. In some implementations, such as the implementation depicted by FIG. 1, the mood evaluation engine can output a confidence score and associate the confidence score with the mood of the user of the user device 110 determined by the mood evaluation engine 132. In such implementations, the present disclosure can determine, at decisioning engine 133, whether the confidence score of the determined mood satisfies a predetermined threshold. If the determined confidence score satisfies the predetermined threshold, then the decisioning engine 133 can determine that the mood evaluation engine determined the likely mood of the user of the user device 110 with a sufficient level of accuracy and the system 100 can provide data 133a identifying a likely mood of the user of the user device 110 to the diagnosis engine 134. Diagnosis as used herein, refers to either a clinical diagnosis or a non-clinical assessment of symptoms. Alternatively, if the determined confidence score does not satisfy the predetermined threshold, then the decisioning engine 133 can determine that the mood evaluation engine did not determine the likely mood of the user of the user device 110 with a sufficient level of accuracy and the system 100 can instruct 133b the conversation engine 131 to generate and provide an additional communication to the user of the user device 110 using stage 711 in order to obtain additional response data from the user that can be used, by the mood evaluation engine 132, to sufficiently determine a likely mood of the user of the user device. The system 100 can continue this iterative feedback loop until the decision engine 133 determines that the mood evaluation engine 132 has sufficiently determined a likely mood of the user of the user device 110. Aspects of this process for the determining a likely mood of user of the user device 110 based on the conversation engine's 131 interaction with the user are discussed in more detail below.

Alternatively, for the instances where the rules-based conversation agent 710 has determined to invoke the LLM 730, the rules-based conversation agent 710 can provide the output data generated by the LLM 730 at stage 732 as an input to the mood evaluation engine 132. In such instances, the mood evaluation engine 132 can be configured to use a rules engine that is configured to map the output data generated by the LLM 730 to a particular mood. For example, the rules engine 132 can map the output (e.g., a problem that the user is experiencing related to anxiety or depression) generated by the LLM 730 at stage 732 to a classification of whether the user's likely mood is a positive mood or a negative mood. In such instances, the mood evaluation 132 can provide 133a indicating a likely mood of the user (e.g., the classification of the user's mood as positive or negative) as an input to the diagnosis engine 134. Then, the diagnosis engine can determine, based on data 133a indicating a likely mood of the user of the user device 110, digital therapeutic content from one or more of database 140, 141, 142 that can be administered to the user of the user device 110 in order to treat one or more symptoms of anxiety or depression. Thus, in implementations where the LLM 730 is invoked by the rules-based conversational agent 730, the determination of therapeutic content by the diagnosis engine 134 is based on the output data generated by the LLM 730.

The above examples describe conversational techniques that can be employed by the an LLM based conversational engine 131 using the flowchart described with reference to FIG. 7 in order to elicit information from the user of the user device 110 that is indicative of the user's likely mood. These conversational techniques are intended to be exemplary in nature and not limiting. In addition, it should be understood that these conversational techniques should not be viewed in isolation and features of each of these conversational techniques can be used across implementations.

In some instances, the user's mood can be classified based on a single communication cycle between the conversation engine 131 and the user of the user device 110, with a conversation cycle comprising a communication from the conversation engine 131 to the user of the user device 110 and a responsive communication from the user of the user device 110 to the conversation engine 131. However, in some implementations as described above, classification of the user's likely mood may be based on conversational techniques that are iterative in nature, thus enabling the conversational engine 131 to establish a therapeutic alliance with the user of the user device 110 that functions to improve adherence of the user to the therapeutic application 112. Once the decisioning 133 has determined, using these conversational techniques described above, a likely mood of the user of the user device 110, the decisioning engine 133 can provide data 133a indicating a likely mood of the user as an input to the diagnosis engine 134.

However, the present disclosure is not limited to the examples described above. For example, in some implementations, the conversation engine 131 can be an LLM-based conversation engine that does not include a corresponding rules-based conversation agent 710. In such implementations, the LLM of the LLM-based conversation engine be configured (e.g., trained) in a manner that enables the LLM to classify communications to the user into a particular mood of the user. For example, the LLM can be configured to engage a user over the course of one or more communication cycles, process the communications sent to the user of the user device 110, the responses received from the user of the user device 110, or both, and then classify the data obtained from these one or more communications cycles into a likely mood classification for the user. For example, in some implementations, the LLM can be configured to classify the data obtained from these one or more communication cycles into a positive mood classification or a negative mood classification. Then, the LLM-based conversation engine can provide the data 133a indicating a likely mood of the user of the user device 110 (e.g., output data generated by the LLM-based conversation engine indicating whether the user's mood is likely positive or likely negative) to the diagnosis engine 134.

In LLM-based conversation engines that do not include a rules-based conversation agent, the LLM can be trained using reinforcement training techniques. In such implementations, the LLM may be trained, for example, using transcripts of therapy sessions between a patient and therapists. The LLM may be tested with training prompts and training response data and used to generated output data based on the training prompts and training response data. The output data generated by the LLM based on processing the training prompts and training response data can be evaluated to determine its level of accuracy. Then, the positive reinforcement or negative reinforcement can be provided to the LLM based on the level of accuracy of the responses that the LLM generated. This reinforcement training process can be iteratively performed until the LLM sufficiently generates output data indicative of a classification of a user's mood that can be provided as data 133a to the diagnosis engine.

Though the example of an LLM-based conversation engine that does not include a rules-based conversation agent 710 is described here is described as being trained to generate output data indicating a user's mood based on the processing of a prompt and response data, the present disclosure is not so limited. Instead, in some implementations, the LLM-based conversation engine that does not include a rules-based conversation agent 710 can be trained to generate other types of output data such as an indication as to whether the user is experiencing symptoms related to anxiety or depression, a problem the user is experiencing related to anxiety or depression, an indication as to whether the user can benefit from an empathetic response, or other types of output data. In such instances, post-processing operations can be implemented in order to map the output of the LLM to the digital therapeutic treatments such as one or more CBTs that can administered to a user according to a dosing schedule determined by the dosing engine.

The diagnosis engine 134 can determine, based on data 133a indicating a likely mood of the user of the user device 110, digital therapeutic content from one or more of database 140, 141, 142 that can be administered to the user of the user device 110 in order to treat one or more symptoms of anxiety or depression. In some implementations, the one or more databases can include a cognitive behavioral therapy (CBT) Tools database 140, an interpersonal psychotherapy (IPT) tools database 141, and anxiety or depression specific database 142, each of which will be described in more detail below.

The CBT tools database 140 can include broad-based cognitive behavioral therapy (CBT) lessons that are configured to treat symptoms that are not necessarily specific to a particular mental health condition (e.g., anxiety, depression, stress, or burnout). In some implementations, for example, CBT lessons stored in the CBT Tools database include broad-based content designed in a manner that, when consumed by an individual exhibiting one or more symptoms of a mental health condition, causes a therapeutic effect on the individual that treats the one or more symptoms of the mental health condition. Though the CBT Tool content stored in CBT tools database can treat the symptoms of one or more mental health conditions, the CBT Tool content is not specifically designed for a particular mental health condition. This means that, by way of example, a therapeutic content module stored in the CBT Tool database 140 may be equally effective for treating an individual exhibiting symptom of anxiety or depression as it would be for a patient exhibiting symptoms of another mental health condition. This may be, e.g., because the therapeutic content may be designed, e.g., to just cheer the individual up without having any explicit content that discusses or describes aspects of anxiety or depression.

In some implementations, these CBT Tools are designed to intervene on cognitions by, e.g., challenging and/or changing automatic thinking patterns and behaviors through behavioral activation. In some implementations, one or more of the respective CBT Tools can be configured to include a psychological assessment, psychoeducation about CBT and aspects of one or more mental health conditions, mood monitoring, practice with CBT tools (e.g., thought challenging), practice sessions, or any combination thereof.

In some implementations, CBT Tools are configured to cause a change in thinking patterns and behaviors through behavioral activation. Behavioral activation encourages participants to engage in positive, pleasurable behaviors in order to manage and/or improve an individual's mood. Behavioral activation CBT Tools can include therapeutic content that triggers the release of chemicals such as, e.g., dopamine, in the brain of an individual viewing the therapeutic content. Such CBT tools that activate such biological responses in an individual impact the individual's mood, with the released chemicals causing an improvement in the individual's mood. Behavioral activation also addresses behavioral avoidance and other barriers to engaging in such behaviors using CBT Tools to teach problem solving techniques and alternative coping strategies, helping people have more regulated and higher functioning lives. Accordingly, CBT Tools utilizing behavioral activation techniques can therapeutically treat an individual exhibiting one or more symptoms of anxiety or depression, which may cause an individual to feel unhappy or insecure, by triggering release of chemicals within an individual's body that counteract those feelings on unhappiness or insecurity by causing the individual to feel happiness, confidence, and positivity.

In some implementations, one or more of the CBT Tools can be configured to interface between the conversation engine 131 and the conversation engine API 116 of the therapeutic application 112, thus enabling interactivity between the user of the user device 110 and the conversation engine 131 within the context of the CBT Tool. In such implementations, the CBT Tool can comprise natural language conversation back and forth between the user and the conversation engine 131, with the conversation engine 131 storing user responses in a memory location that is associated with the user's user of the CBT Tool. In such instances, the conversation engine 131 can draw on the user's previous responses during prior therapeutic treatments using the CBT Tool during subsequent therapeutic treatments using the CBT Tool, as the conversation engine 131 can recall prior interactions with the user using the CBT Tool based on the stored responses.

The Interpersonal Psychotherapy (IPT) Tool database 141 can include psychotherapy lessons that provide intervention that can be used to treat anxiety or depression. In particular, IPT tools work to alleviate clinical symptomatology by helping an individual improve interpersonal functioning. IPT has been proven to benefit mental health conditions such as depression based in attachment and interpersonal theory postulates that depressive symptomatology has biopsychosocial roots, and as a result, consequences of interpersonal distress fall into one of a plurality of predefined problem areas including role disputes, role transitions, grief, and interpersonal deficits. Like the content in the CBT Tool database 140, though the IPT Tools in IPT Tool database 141 can be used to treat symptoms of anxiety or depression, each IPT Tool need not necessarily be specific to a particular mental health condition, and can provide emotional support and help live better without bothersome symptoms of depression or anxiety.

The anxiety or depression (A/D) specific database 142 can include psychotherapy lessons that are specifically tailored to address symptoms of anxiety or depression. In contrast to the CBT Tools database and IPT Tools database, which each include content in the form of CTB Tools or IPT Tools that are designed independent of a particular mental health condition, A/D database 142 includes content that are designed specifically to treat one or more particular symptoms of anxiety or depression. For example, the A/D specific lessons database 142 may include therapeutic content that has been specifically designed to treat symptoms related to anxiety or depression using lessons designed to improve a person's mood and have explicit discussions and/or teachings related to the topics of anxiety or depression.

The example of FIG. 1 describes the database 142 as being an anxiety or depression (A/D) specific database 142, the present disclosure is not so limited. Instead, for example, the database 142 should be understood to be a database storing condition-specific psychotherapy lessons. The example of FIG. 1 describes treatment anxiety or depression. Thus, the condition-specific psychotherapy database 142 is an anxiety or depression (A/D) database.

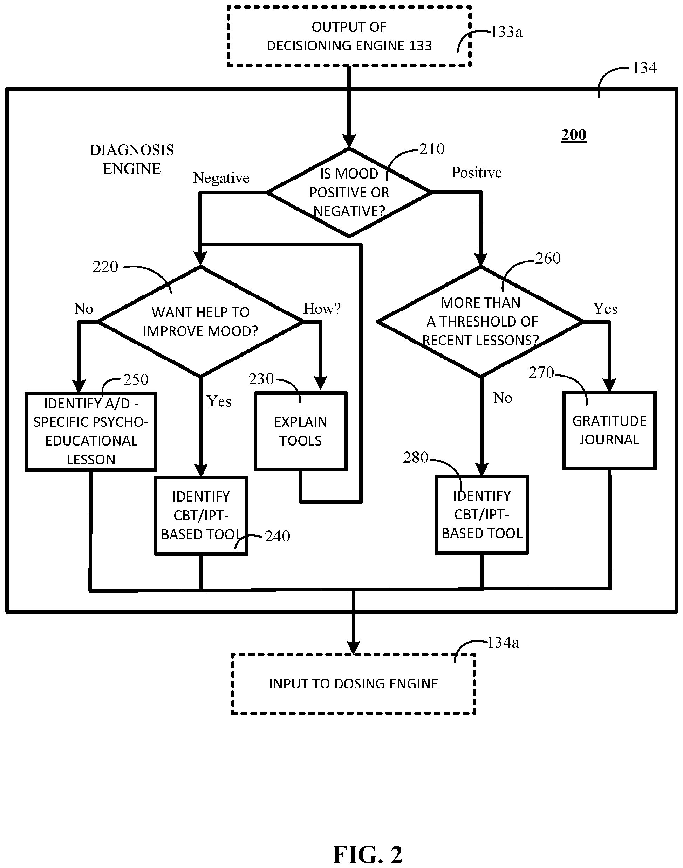

The execution of the system 100 is further discussed with reference to FIG. 2, which is a flowchart of an example of a process 200 that can be executed by a diagnosis engine 131, in accordance with one aspect of the present disclosure. As shown in FIG. 2 (and FIG. 1), execution of the system 100 can continue with the decisioning engine 133 providing data 133a indicating a likely mood of the user of the user device 110 to the diagnosis engine 134. The diagnosis engine 134 can use a decisioning engine 210 to determine whether the data 133a indicating a likely mood of the user indicates a positive mood or a negative mood. If the decisioning engine 210 determines that the data 133a indicates that the mood of the user of the user device 110 is negative, then the execution of the process 200 can continue to decisioning engine 220.

Here, the decisioning engine 220 can instruct the conversation engine 131 to ask the user of the user device 110 if the user wants help improving the user's mood. In one case, the user of the user device 110 may submit a response that asks the conversation engine 132 how the therapeutic application 112 can help to improve the user's mood. In such instances, the decisioning engine 220 of the dosing engine 134 can instruct the conversation engine 132 to explain 230 the therapeutic tools that can be provided by the therapeutic application 112. Such an explanation may include, e.g., a description and/or tutorial regarding anxiety or depression specific psychoeducational lessons, a description and/or tutorial regarding cognitive behavioral therapy (CBT) based tools, a description and/or tutorial regarding interpersonal theory (IPT) based tools, or any combination thereof. In some implementations, the conversation engine 131 can provide links, e.g., to tutorial content that describes each of the therapeutic tools of anxiety or depression specific psychoeducational lessons, CBT based tools, and/or IPT based tools. In such instances, the user has the discretion to view the tutorials for one or more of the therapeutic tools or emotional support.

During the execution of the process 200 performed by the diagnosis engine 134 within the context of the system of FIG. 1, a second case can occur during operation of the decisioning engine 220. For example, in some instances, a user may respond to the prompting for help with his/her mood by indicating that, yes, the user would like help improving the user's mood. In such instances, the decisioning engine 220 of the diagnosis engine 134 can identify 240 one or more CBT and/or IPT based tools stored in the CBT Tools DB 140 or IPT Tools DB 141. In some implementations, the identification of the CBT Tools and/or IPT Tools by the diagnosis engine 134 can be based on the diagnosis engine's 134 analysis of the response data received from the user during the user's interaction with the conversation engine 131. As described above, response data can include (i) a single natural language response, (ii) multiple natural language responses, (iii) data indicating selections of one or more pre-programmed buttons corresponding to different emotional states (e.g., moods), (iv) or a combination thereof.

During the execution of the process 200 performed by the diagnosis engine within the context of the system of FIG. 1, a third case can occur during operation of the decisioning engine 220. For example, in some instances, a user may respond to the prompting for help with his/her mood by indicating that, no, the user would not like help improving the user's mood. In such instances, the decisioning engine 220 of the diagnosis engine 134 can identify 250 one or more anxiety or depression (A/D) specific psychoeducational lessons stored in the anxiety or depression (A/D) specific lesson database 142. In some implementations, the identification of the anxiety or depression (A/D) specific psychoeducational lessons by the diagnosis engine 134 can be based on the diagnosis engine's 134 analysis of the response data received from the user during the user's interaction with the conversation engine 131. As described above, response data can include (i) a single natural language response, (ii) multiple natural language responses, (iii) data indicating selections of one or more pre-programmed buttons corresponding to different emotional states (e.g., moods), (iv) or a combination thereof. In other implementations, the diagnosis engine may already have existing information such as, e.g., a user profile, indicating one or more symptoms of anxiety or depression a user is exhibiting and identify anxiety or depression specific psychoeducational lessons for the user based on the existing information.

The above example describes a scenario where the diagnosis engine 134 is being used to diagnosis treatment for anxiety or depression. Thus, in the example of FIG. 2, when the user's mood is determined at decisioning engine 210 to be negative and the decision engine 220 determines that the user wants help improving the user's mood, then the diagnosis engine 250 identify anxiety or depression (A/D) specific psychotherapy lessons. However, the present disclosure is not so limited. Instead, the diagnosis engine at 250 can identify condition-specific treatment (whereas treatment at stage 240 or 280 is not condition specific).

The above operations of the diagnosis engine 131 are performed by executing the operations described by the process 200 when the decisioning engine 210 of the diagnosis engine 134 determines that the likely mood of the user of the user device 110 is negative. Alternatively, however, the decisioning engine 210 can initiate performance of different operations responsive to a determination, by the decisioning engine 210, that the data 133a indicates that the user of user device 110 has a mood that is likely positive.

Here, the decisioning engine 210 can determine that the data 133a indicates that the likely mood of the user is positive. Based on a determination that the likely mood of the user of user device 110 is positive, the decisioning engine 210 can instruct the decisioning engine 260 to evaluate the number of recent lessons completed by the user of the user device 110. In a first case, the decisioning engine 260 can determine that the user of the user device 110 has recently consumed (e.g., watched therapeutic content, listened to therapeutic content, read therapeutic content, or a combination thereof) more than a threshold number of recent therapeutic lessons. Based on a determination that the user of the user device 110 has recently completed more than a threshold number of therapeutic lessons, the decisioning engine 260 of the diagnosis engine can cause the therapeutic application 112 to display a gratitude journal in the display of the therapeutic application 112. The gratitude journal provides, e.g., the user of the user device 110 a mechanism to log the user's positive thoughts in a journal, the action of which may reinforce the user's positive feelings. Alternatively, if the decisioning engine 260 determines that the user of the user device has not recently completed more than a threshold number of therapeutic lessons, the decisioning engine 260 of the diagnosis engine 200 can identify 280 one or more CBT and/or IPT based tools stored in the CBT Tools DB 140 or IPT Tools DB 141. For purposes of this disclosure, "recently" can include a threshold period of time that precedes a point in time when the decisioning engine 260 of the diagnosis engine 134 evaluates the number of therapeutic lessons the user has completed. The threshold period of time can include a predetermined number of hours, days, weeks, or the like. In some implementations, the identification of the CBT Tools and/or IPT Tools by the diagnosis engine 134 can be based on the diagnosis engine's analysis of the response data received from the user during the user's interaction with the conversation engine 131. As described above, response data can include (i) a single natural language response, (ii) multiple natural language responses, (iii) data indicating selections of one or more pre-programmed buttons corresponding to different emotional states (e.g., moods), (iv) or a combination thereof.

The output data 134a of the diagnosis engine 134 is data that identifies one or more therapeutic content items that are to be administered to the user. For purposes of this disclosure, data that identifies one or more therapeutic content items that are to be administered to the user of the user device 110 can include data that references the one or more therapeutic content items (e.g., one or more URLs), data that indicates the one or more therapeutic content items (e.g., data that the user device 110 can use to identify or unlock therapeutic content), or one or more therapeutic content media files.

Data that references one or more therapeutic content items can be processed by the user device 110 or selected by the user of the user device 110 in order to access the therapeutic content items that are to be administered to the user of the user device 110. Such reference data may link to therapeutic content items that are stored locally on the user device 110 or remotely from a user device 110. Data stored remotely from the user device 110 may be stored in one or more databases such as, e.g., database 140, 141, 142. In some implementations, data that references one or more therapeutic content items can include, for example, a URL.

Data that indicates one or more therapeutic content items can be processed by a user device 110 in order to instruct the user device as to a particular set of one or more therapeutic content items that are to be administered to a user of the user device 110. In some implementations, data that indicates one or more therapeutic content items can include, for example, a content identifier for a therapeutic content item or group of one or more therapeutic content items. In such implementations, the therapeutic application 112 on the user device 110 may have access to a library of therapeutic content items stored locally on the user device 110 or remotely from the user device 110 that is accessible by the user device 110. Then, in such implementations, the user device 110 may use the data indicating the one or more therapeutic content items to identify one or more particular therapeutic content items from the library of therapeutic content items and administer the one or more identified particular therapeutic content items to the user of the user device 110.

In some implementations, data that indicates one or more therapeutic content items can include an authorization key, a security key, private key, a license, or the like for one or more therapeutic content items. In such implementations, the therapeutic application 112 on the user device 110 may have access to a library of therapeutic content items that are stored locally on user device 110 or remotely from the user device 110. However, in this implementation, the one or more therapeutic content items accessible by the therapeutic application 112 of the user device 110 may have restricted access. Restricted access can include content that is access controlled, encrypted, or otherwise protected. In such implementations, receipt of a data indicating one or more particular therapeutic content items such as an authorization key, security key, private key, license, or the like gives the therapeutic application 112 of the user device 110 the authority to access and unlock the particular therapeutic content items that correspond to the received indicating data. Such an implementation provides the benefit of prohibiting a user of a user device 110 from accessing therapeutic content items that were not identified by the diagnosis engine 200 to treat the user of the user device 110 (or otherwise prescribed for the user).

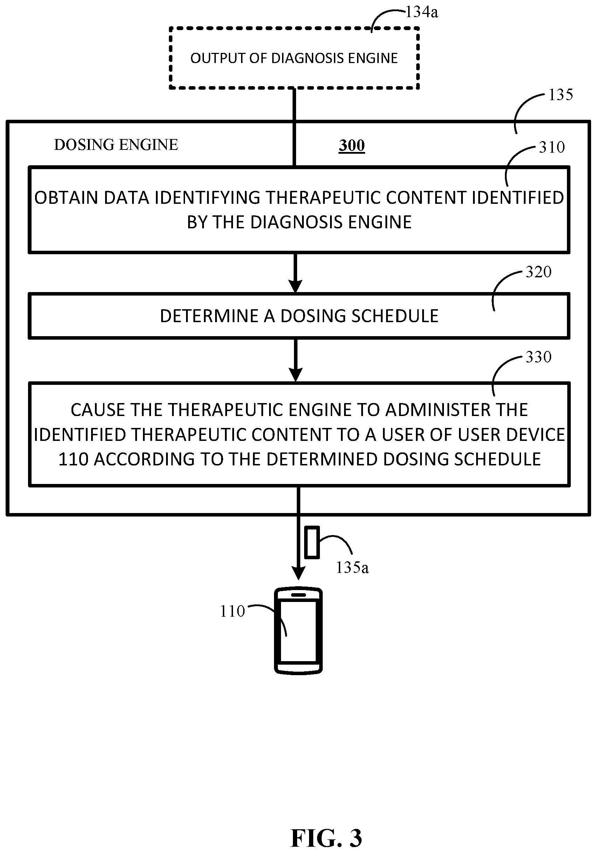

The diagnosis engine 134 can provide this output data 134a to the dosing engine 134a. The operations performed by the dosing engine 134a within the context of the system of FIG. 1 are described with reference to the process of FIG. 3. With reference to FIG. 3, the dosing engine 135 can obtain 310 output data 134a of the diagnosis engine 134 that identifies one or more therapeutic content items that are to be administered to a user of the user device 110. The one or more therapeutic content items may include one or more CBT Tools, one or more IPT tools, or one or more anxiety or depression (A/D) specific lessons.

The dosing engine can determine 320 a dosing schedule for the user. The dosing schedule can indicate a frequency of treatment, a duration of treatment, or both. A frequency of treatment indicates a number of treatments per specific time period (e.g., hour, day, week, or month, or the like). A duration of treatment indicates how many time periods the treatment is to be administered (e.g., 10 days, 2 weeks, 8 weeks, 2 to 8 weeks, at least 8 weeks, 3 months, 1-3 months, or the like). Thus, by way of example, a dosing schedule determined by the dosing engine can be for the user to consume the prescribed content for at least 3 times per week for a duration of two to eight weeks. However, other frequencies of treatment and durations of treatment can be prescribed by the dosing engine 135 based on a conversation thread between the user of the user device 110 and the conversation engine 131, the user's mood as determined by an analysis of a conversation thread between the user of the user device and the conversation engine 131, or the like. In some implementations, a persistent symptom mitigation engine 136 may provide information about a user's treatment to the diagnosis engine 135 and request that the dosing engine 135 determine if the user's dosing schedule is to be adjusted. For example, based on feedback information obtained by the persistent symptom mitigation engine 136, the dosing engine 136 can increase a frequency of treatment, decrease a frequency of treatment, increase a duration of treatment, decrease a duration of treatment, or any combination thereof. Modifications to an existing dosing schedule can be made, by the dosing engine 135, based on, e.g., symptoms of a mental health condition detected by the persistent symptom mitigation engine, a number of recent lessons completed, trending emotional behavior responsive to treatment determined based on user feedback, or the like.

In some implementations, the dosing engine 135 can determine that a longer, initial duration of treatment is beneficial to a user of the user device 110. An example of a longer duration treatment prescribed by the dosing engine 135 may include, for example, a dosing schedule of 3 times a week for at least 8 weeks. Though the examples used herein describe a frequency of 3 times per time period for a duration of treatment, the present disclosure is not so limited. Instead, the dosing engine 135 may determine any frequency of treatment per time period. The therapeutic application 112 can be configured to monitor the user's adherence to the specified dosing schedule and intervene to the extent the user strays from the dosing schedule. For example, upon determination that the user has strayed from the dosing schedule, the conversation engine 131 can trigger an accountability conversation with the user to get the user back on schedule, explain a paradoxical injunction (e.g., a paradoxical cost benefit analysis), or the like.

The dosing engine can cause 330 the therapeutic engine 118 of the therapeutic application 112 to administer the therapeutic content to the user of the user device 110 according to the determined dosing schedule. This can include, for example, transmitting the therapeutic regimen data 135a to the user device 110. The therapeutic regimen data 135a can include (i) the obtained therapeutic content, data that references the therapeutic content, and/or data that indicates the therapeutic content, and (ii) the dosing schedule determined at stage 320. The data 135a can also include instructions that cause the triggering engine 114 to alert the user of the user device 110 as to the receipt of the therapeutic regimen data 135a in order to trigger initiation of the administration of treatment. Alternatively, in other implementations, the dosing engine 135, or other component of system 100 such as the conversation engine 131, can transmit a separate notification (e.g., a push notification) to the user device 110 that causes the triggering engine 114 to alert the user of the user device 110 to the receipt of the therapeutic regimen data 135a. In this alternative implementation, the dosing schedule 135a can, but is not required to, include data that alerts the user of the user device 135a regarding the receipt of the therapeutic regimen data 135a.

Alternatively, in the case of the therapeutic content being the gratitude journal, the dosing engine 135 can obtain data indicating how to access the gratitude journal, determine a dosing schedule indicating a frequency with which the user of the user device 110 is to write in the gratitude journal, and then cause the treatment engine to administer the treatment that includes instructions, to the user, to add an entry to the gratitude journal in accordance with the frequency specified by the dosing schedule. The therapeutic application 112 can be configured to monitor the user's adherence to the specified dosing schedule and intervene to the extent the user strays from the dosing schedule. For example, upon determination by the therapeutic application 112 that the user has strayed from the dosing schedule, the conversation engine 131 can be informed of the user straying and then trigger an accountability conversation with the user to get the user back on schedule, explain a paradoxical injunction (e.g., a paradoxical cost benefit analysis), or the like.

The user device 110 can receive the therapeutic regimen data 135a from server 130. Based on the receipt of the therapeutic regimen data 135a or an accompanying notification, the triggering engine 114 can alert the user of the user device 110 regarding the receipt of the therapeutic regimen data 135a and prompt the user of the user device 110 to check in for administration of the therapeutic treatment. In some implementations, the user can check-in responsive to the receipt of the notification indicating that the therapeutic regimen has been received and begin treatment. In other implementations, the therapeutic application 112 may provide the user with an option to delay start of the therapeutic treatment in a manner that is consistent with the therapeutic regimen data 135. This means that, by way of example, if the therapeutic regimen 135a requires that the user consume one or more therapeutic content items for 2 times per week for a certain duration of weeks, then the therapeutic application will only permit the user of the user device up to 5 days of delay so that the user still has 2 days remaining in the week to complete the treatment for that week. In some implementations, if the user does not adhere to the dosing regimen 135a, the therapeutic application 113 can be configured to intervene in a manner that causes the user to get back on the dosing regimen. For example, in some implementations, upon determination by the therapeutic application 112 that the user has strayed from the dosing schedule of a dosing regimen, the conversation engine 131 can be informed of the user straying and then trigger an accountability conversation with the user to get the user back on schedule, explain a paradoxical injunction (e.g., a paradoxical cost benefit analysis), or the like.

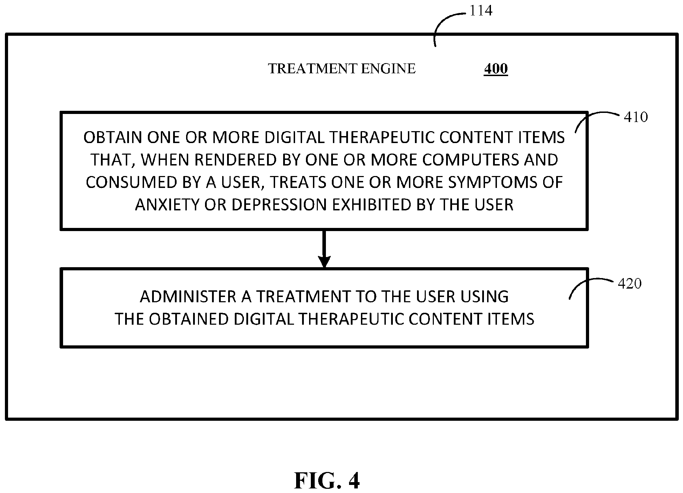

The therapeutic application 112 can use the therapeutic engine 118 to administer a therapeutic treatment to the user of the user device 110 using the one or more therapeutic content items after receipt of the therapeutic regimen 13a. The process for using the therapeutic engine 118 to administer a therapeutic treatment to the user of the user device is described with reference to FIG. 4. With reference to FIG. 4, the therapeutic engine 118 can begin execution of the process 400 by obtaining (410) one or more digital therapeutic content items identified by the therapeutic regime data 135a. This digital therapeutic content includes, for example, one or more one or more CBT Tools, one or more IPT tools, or one or more anxiety or depression specific lessons. These digital therapeutic content items, when rendered by the user device 110 and consumed by a user, treats one or more symptoms of the user's anxiety or depression.

The therapeutic engine 118 can obtain 410 the one or more digital therapeutic content items in a number of different ways based on the manner in which the therapeutic regimen data 135a identifies the one or more digital therapeutic content items. For example, if the therapeutic regimen data 135a includes a reference such as a URL to one or more digital therapeutic content items, the therapeutic engine 118 can obtain the URL, access the URL, and obtain the one or more digital therapeutic content items referenced by the URL. Alternatively, if the therapeutic regimen includes data indicating one or more digital therapeutic content items, then the therapeutic engine 118 can use the data indicating the one or more digital therapeutic content items to access one or more digital therapeutic content items using the data from the therapeutic regimen data that indicates the one or more digital therapeutic content items. Data indicating the therapeutic content item can include, for example, a content identifier, authorization key, security key, private key, license, or the like.

The therapeutic engine 118 can administer 420 a treatment to the user using the obtained one or more digital therapeutic content items. In some implementations, administering the treatment can include rendering, by the user device 110, the one or more obtained digital therapeutic content items on a graphical user interface of the user device 110 when the user is viewing the graphical user interface, a predetermined number of times a week for a duration of two to eight weeks. In some implementations, the predetermined number of times is three times per week.

In some implementations, the therapeutic application 112 can employ various techniques to determine whether a user is viewing the graphical user interface of the user device 110 when the one or more digital therapeutic content items are being rendered. For example, in some implementations, the therapeutic application 112 can periodically prompt or randomly prompt the user to select the display of a graphical element that was silently displayed on the graphical user interface of the user device 110. If the user fails to select the displayed graphical element, the therapeutic application 112 can determine that the user is not viewing the graphical user interface of the user device 110 and perform one or more operations (e.g., alerting user with an audible alert, alerting user with a haptic feedback alert, or the like) in order to direct the user's attention back to the digital therapeutic content item being rendered on the graphical user interface of the user device 110.

In some implementations, the therapeutic application 112 can obtain and interpret sensor data of the user device 110 in order to determine whether the user of the user device 110 is viewing the graphical user interface of the user device 110. For example, the therapeutic application 112 can determined based on, for example, sensor data from a gyroscope, accelerometer, or both, the orientation of the user device 110 and whether the current orientation of the user device 110 is an orientation in which the user is likely to hold the user device 110 when looking at the graphical user interface of the user device 110. Another example can include the user device 110 periodically or randomly capturing images from a user-facing camera of the user device 110. In such instances, the therapeutic application 112 can perform image analysis on the captured images to determine if the user is currently looking at the graphical user interface of the user device 110. If the therapeutic application 112 determines that the user is not viewing the graphical user interface of the user device 110, the therapeutic application can perform one or more operations (e.g., alerting user with an audible alert, alerting user with a haptic feedback alert, or the like) in order to direct the user's attention back to the digital therapeutic content item being rendered on the graphical user interface of the user device 110.

Other, optional, features may be employed by the system 100. For example, in some implementations, the system 100 can be modified to include optional features such as the crisis mitigation. In such alternative implementations, the mood evaluation engine 132 can be configured to evaluate response data from the user device 110 to detect whether a predetermined level of depression, anxiety, or emotional crisis is present in the user's responses. Response data can include, for example, a single natural language response, multiple natural language responses, a conversation thread, data indicating selection of one or more pre-programmed buttons, or any combination thereof. Based on a determination, by the mood evaluation engine 132, that a predetermined level of depression, anxiety, or emotional crisis has been detected in the user's response data, the mood evaluation engine 132 can instruct the conversation engine 131 to perform an intervention with the user. In some implementations, the intervention can include natural language communication from the conversation engine 131 that is designed to improve the user's mood. Alternatively, or in addition, the intervention can include the conversation engine providing links to content that, when consumed by the user, causes the user's mood to improve. Alternatively, or in addition, the intervention can include the conversation engine 131 alerting an emergency contact, police department, or fire department in an effort to cause the emergency contact to intervene.

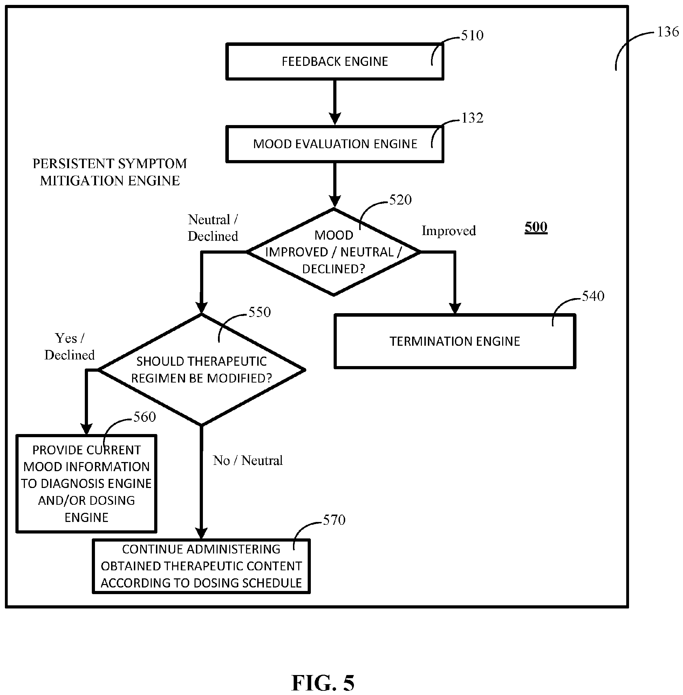

FIG. 5 is a flowchart of an example of a process 500 that can be executed by a persistent symptom mitigation engine 136, in accordance with one aspect of the present disclosure. Execution of the process 500 by the persistent symptom mitigation engine 136 utilizes the mood evaluation engine 132 of FIG. 1.

The persistent symptom mitigation engine 136 can use a feedback engine 510 to obtain feedback from a user of the user device 110 regarding therapeutic treatment received. In some implementations, the feedback engine 510 can transmit a message 136a to the user of the user device 110 that can be accessed via the therapeutic application 112. This message can include, for example, a survey of one or more questions in the form of an email message, text message, or the like. In such implementations, the feedback engine 510 can await receipt of the user's responses to the survey of one or more questions before continuing execution of the process 500. Alternatively, in some implementations, the feedback engine 510 can instruct the conversation engine 131 to interact with the user of the user device 110 in order to elicit feedback regarding the therapeutic treatment from the user of the user device 110. Then, the conversation engine 131 can provide all, or a portion of, the conversation thread to the feedback engine 510 for processing in the remainder of process 500.

The feedback engine 136 can receive feedback data 131d and provide the feedback data as an input to the mood evaluation engine 132. The feedback data 131d can include a response to the survey of one or more questions from the feedback engine 510. Alternatively, or in addition, the feedback data 136a can include all, or a portion of, a conversation thread between the user of the user device and the conversation engine 131. The mood evaluation engine can process the feedback data 131d to determine a current mood of the user of the user device 110, a trending direction of the user's mood, or a combination thereof. The mood evaluation engine 132 can generate output data indicating a current mood of the user of the user device 110, a trending direction of the user's mood, or a combination thereof. The mood evaluation engine 132 can provide the generated output data as an input to the decisioning engine 520.

The decisioning engine 520 can determine whether the user's mood has improved, declined, or stayed neutral. If the decisioning engine 520 determines that the user's mood has improved by a threshold amount, then the decisioning engine 520 can instruct the termination engine 540 to terminate the remainder of the user's treatment specified by the therapeutic regimen data. Alternatively, if the decisioning engine 520 determines that the user's mood is either neutral or has declined, then the decisioning engine 520 can continue execution of the process 500 by instructing the decisioning engine 550 to determine whether the user's therapeutic regimen should be modified.

The decisioning engine 550 can determine whether the user's therapeutic treatment regimen should be modified. If the decisioning engine 550 determines that the user's mood has declined, then the decisioning engine can provide 560 current mood information to the diagnosis engine 134 or the dosing engine 135 in order to have the user's therapeutic treatment modified. In implementations, for example, where decisioning engine 550 provides 560 information to the diagnosis engine 134, the diagnosis engine 134 can identify therapeutic content based on the current mood information and/or content of feedback data 131d and then provide the identified therapeutic content to the dosing engine 135. Alternatively, the decisioning engine 550 can determine that user's mood is neutral. In such instances, the decisioning engine 550 can determine that the system 100 should continue 570 administering the previously obtained therapeutic content according to the dosing schedule established by the therapeutic regimen data.

Alternatively, or in addition, in implementations, for example, where the decisioning engine 550 provides 560 current mood information to the dosing engine 135, the dosing engine 135 can, based on feedback information obtained by the persistent symptom mitigation engine 136, increase a frequency of treatment, decrease a frequency of treatment, increase a duration of treatment, decrease a duration of treatment, or any combination thereof. Modifications to an existing dosing schedule can be made, by the dosing engine 135, based on, e.g., symptoms of a mental health condition detected by the persistent symptom mitigation engine, a number of recent lessons completed, trending emotional behavior responsive to treatment determined based on user feedback, or the like.

FIG. 6 is a diagram of an example of a user device 610 that is configured to generate, dose, and administer a digital therapeutic to treat one or more symptoms related to anxiety or depression. Regarding the user device 610, the user device 610 is similar to the user device 110, except the user device 510 is configured to execute the entirety of system 100 within the user device. Specifically, this means that in addition to the triggering engine 114, the conversation engine API, and the therapeutic engine 118, the user device 510 also includes the conversation engine 131, the mood evaluation engine 132, the decisioning engine 133, the diagnosis engine 134, the dosing engine 135, and the persistent symptom mitigation engine 136. Each of these respective engines performs the same operations described with reference to system 100, but in the implementation of FIG. 6, are installed on the user device 610 and, thus, reside locally on the user device 610. Likewise, the database 140, 141, 142 are also depicted as residing locally on the user device 610. And, in some implementations, the databases 140, 141, and 142 may reside locally on the user device 610. However, for purposes of the present disclosure, there is no requirement that the database 140, 141, 412 reside locally on the user device 610 and, instead, can be stored remotely and accessed by the user device 610 via one or more networks.

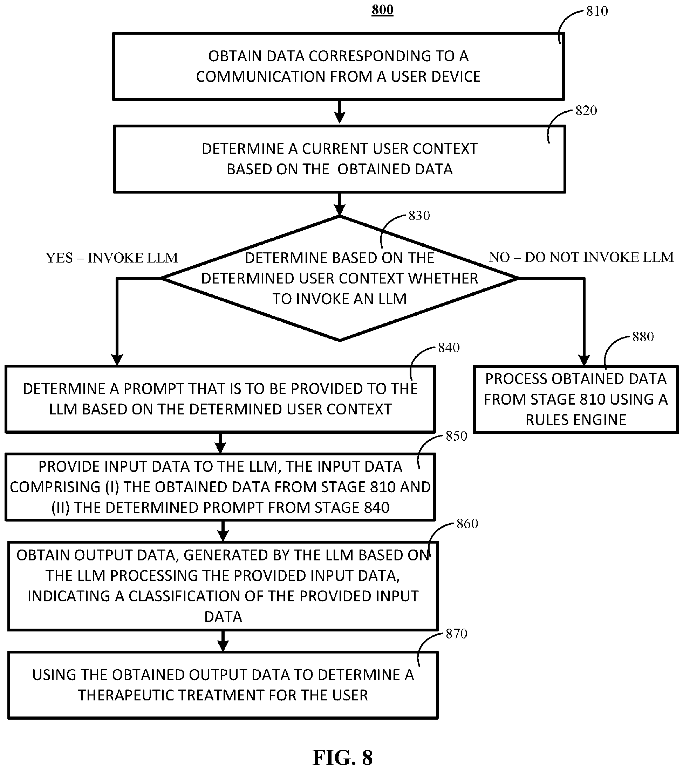

FIG. 8 is a flowchart of a process 800 for determining whether to invoke a large language model (LLM) to treat a mental health disorder, in accordance with one aspect of the present disclosure. For purposes of this disclosure, the process 800 will be described as being performed by a server such as the server 130 of FIG. 1. However, the process 800 can be performed by any computer such as the user device 610 of FIG. 6.

A server can begin execution of the process 800 by using one or more computers to obtain data corresponding to a communication from a user device (810).

The server can continue execution of the process 800 by using one or more computers to determine a current user context based on the obtained data (820).

The server can continue execution of the process 800 by determining, by one or more computers, based on the determined user context, whether to invoke the LLM (830). If, at stage 830, the server determines to invoke the LLM, the server can continue execution of the process 800 at stage 840 by using one or more computers to determine a prompt that is to be provided to the LLM based on the determined user context (840).

The server can continue execution of the process 800 by using one or more computers to provide input data to the LLM, the input data comprising (i) the obtained data and (ii) the determined prompt (850).

The server can continue execution of the process 800 by using one or more computers to obtaining output data, generated by the LLM based on the LLM processing the provided input data, indicating a classification of the provided input data (860).

The server can continue execution of the process 800 by using the obtained output data to determine a therapeutic treatment for the user (870).

In some implementations, the server can determine at stage 830 to not invoke the LLM. In such instances, the server can continue execution of the process 800 after stage 830 by processing the data obtained at stage 810 corresponding to a communication from a user device using a rules engine (880).

In some implementations, the obtained data corresponding to a communication from a user device comprise data indicating a natural language communication.

In some implementations, the obtained data corresponding to a communication from a user device comprise data indicating selection of a preprogrammed button, an emoji, an image, or a video.

In some implementations, the determined user context is data indicating a current user status related to the user's interaction with a therapeutic application.

In some implementations, the determined user context is indicative a history of user interaction with a therapeutic application.

In some implementations, the output data indicating a classification of the provided input data generated by the LLM based on the LLM processing the provided input data.

In some implementations, the LLM has been configured using rule-example pairs.

In some implementations, the rule-example pair comprises a rule that the LLM is configured to follow and an example of the rule the LLM is configured to follow.

In some implementations, the rule-example pair configures the LLM with limits in the communication the LLM can output to the user device for review by the user.

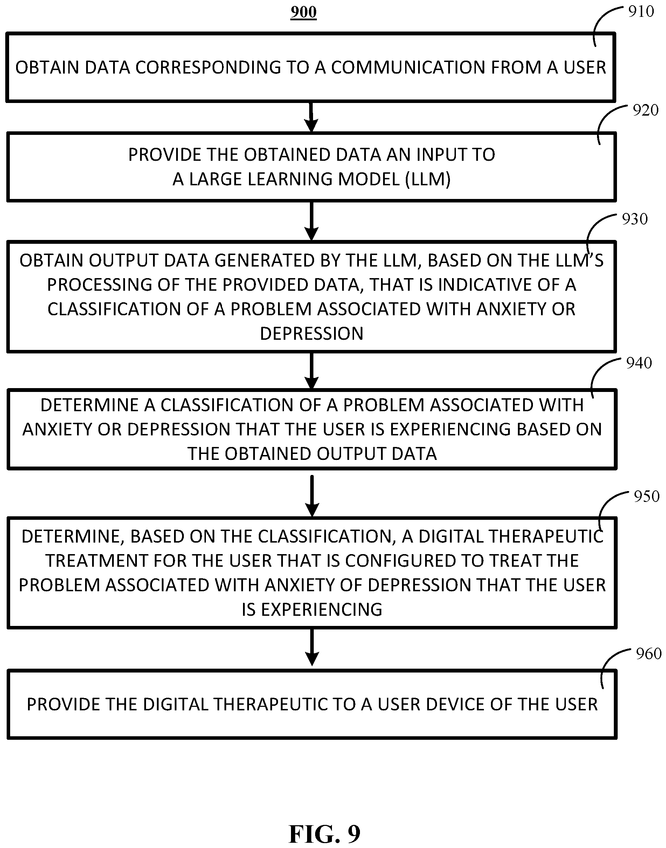

FIG. 9 is a flowchart of a process 900 for using an LLM to classify a problem a user is experiencing related to anxiety or depression, in accordance with one aspect of the present disclosure. For purposes of this disclosure, the process 900 will be described as being performed by a server such as the server 130 of FIG. 1. However, the process 900 can be performed by any computer such as the user device 610 of FIG. 6.

A server can begin execution of the process 900 by using one or more computers to obtain data corresponding to a communication from a user (910).

The server can continue execution of the process 8080 by using one or more computers to provide the obtained data as an input to a large language learning model (LLM) that has been configured to classify input data corresponding to a communication from a user into a category corresponding to a problem associated with anxiety or depression (920).

The server can continue execution of the process 900 by using one or more computers to obtain output data generated by the LLM, based on the LLM's processing of the provided data, that is indicative of a classification of a problem associated with anxiety or depression (930).

The server can continue execution of the process 900 by using one or more computers to determine a classification of a problem associated with anxiety or depression that the user is experiencing based on the obtained output data (940).

The server can continue execution of the process 900 by using one or more computers to determine, based on the determined classification, a digital therapeutic treatment for the user that is configured to treat the problem associated with anxiety or depression that the user is experiencing (950).

The server can continue execution of the process 900 by using one or more computers to providing the digital therapeutic to a user device of the user (960).

In some implementations, the LLM has been configured using rule-example pairs.

In some implementations, the rule-example pair comprises a rule that the LLM is configured to follow and an example of the rule the LLM is configured to follow.

In some implementations, the rule-example pair configures the LLM with limits in the communication the LLM can output to the user device for review by the user.

In some implementations, the problem associated with anxiety or depression comprises at least one of a relationship problem, procrastination, grief, sleep, financial, a major sickness, a minor sickness, loneliness, addiction, pain, anxiety, depression, anger, guilt, regret, or corona.

In some implementations, the server can perform determining a classification of a problem associated with anxiety or depression that the user is experiencing based on the obtained output data can by using one or more computers to map the output generated by the LLM to a classification that indicates a classification of a problem associated with anxiety or depression.

In some implementations, the mapping is performed using a rules engine.

In some implementations, the server can perform determining, by one or more computers, a classification of a problem associated with anxiety or depression that the user is experiencing based on the obtained output data by using one or more computers to evaluate, natural language output generated by the LLM, and based on an evaluation of the natural language output generated by the LLM, determining that the user is in need of emotional support.

In some implementations, execution of the process 900 can include the server using one or more computers to obtain second data indicating a current context associated with the user, and determine a prompt that is to be provided to the LLM based on the determined user context. In such implementations, the server can perform providing the obtained data as an input to a large language learning model (LLM) that has been configured to classify input data corresponding to a communication from a user into a category corresponding to a problem associated with anxiety or depression by using one or more computers to provide the obtained data, the obtained second data, and the obtained prompt as an input to a large language learning model (LLM) that has been configured to classify input data corresponding to a communication from a user, a current user context, and a prompt into a category corresponding to a problem associated with anxiety or depression.

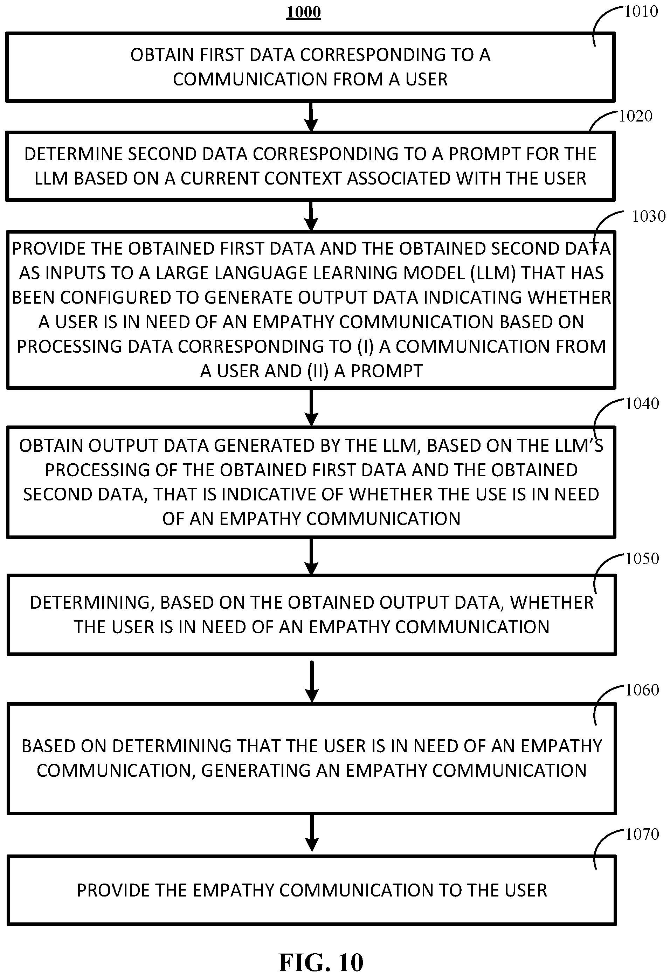

FIG. 10 is a flowchart of a process 1000 for providing empathy to a user based on classification of a user communication by an LLM, in accordance with one aspect of the present disclosure. For purposes of this disclosure, the process 1000 will be described as being performed by a server such as the server 130 of FIG. 1. However, the process 1000 can be performed by any computer such as the user device 610 of FIG. 6.

A server can begin execution of the process 1000 by using one or more computers to obtain first data corresponding to a communication from a user (1010).

The server can continue execution of the process 1000 by using one or more computers to determine second data corresponding to a prompt for the LLM based on a current context associated with the user (1020).

Th server can continue execution of the process 1000 by using one or more computers to provide the obtained first data and the obtained second data as inputs to a large language learning model (LLM) that has been configured to generate output data indicating whether a user is in need of an empathy communication based on processing data corresponding to (i) a communication from a user and (ii) a prompt (1030).

The server can continue execution of the process 1000 by using one or more computers to obtain output data generated by the LLM, based on the LLM's processing of the obtained first data and the obtained second data, that is indicative of a whether the user is in need of an empathy communication (1040).

The server can continue execution of the process 1000 by using one or more computers to determine, based on the obtained output data, whether the user is in need of an empathy communication (1050).

In response to a determination, by one or more computers of the server, that the user is in need of an empathy communication, the server can continue execution of the process 1000 by using one or more computers to generating an empathy communication. Alternatively, if it is determined, by one or more computers of the server, that the user is not in need of an empathy communication, the server can perform one of at least two different operations. In a first implementation, the server may use one or more computers to terminate execution of the process 1000. In a different, second, implementation, the server may use one or more computers to determine if additional communication with the user is necessary as described with reference to FIGs. 1 and 7.

The server can continue execution of the process 1000 by using one or more computers to provide the empathy communication to a user device of the user.

In some implementations, the LLM has been configured using rule-example pairs.

In some implementations, the rule-example pair comprises a rule that the LLM is configured to follow and an example of the rule the LLM is configured to follow.

In some implementations, the rule-example pair configures the LLM with limits in the communication the LLM can output to the user device for review by the user.

In some implementations, the empathy communication is a communication indicating a communication to the user of the user device that acknowledges the user's emotional state related to anxiety or depression and offers emotional support.

In some implementations, determining, by one or more computers of the server, whether the user is in need of emotional support can include using one or more computers of the server to map the output generated by the LLM to a classification that indicates whether the user is in need of emotional support.

In some implementations, the mapping is performed using a rules engine.

In some implementations, the server can determine whether the user is in need of emotional support by using one or more computers to evaluate natural language output generated by the LLM, and then based on an evaluation of the natural language output generated by the LLM, the server can continue execution of the process 1000 by using one or more computers to determine that the user is in need of emotional support.

In some implementations, the server can also provide the current user context as an input to the LLM. In such implementations, execution of the operation of providing providing stage 1030 by the server can include the server using one or computers to provide the obtained first data, the obtained second data, and the current user context as inputs to a large language learning model (LLM) that has been configured to generate output data indicating whether a user is in need of an empathy communication based on processing data corresponding to (i) a communication from a user, (ii) prompt, and (iii) a current user context.

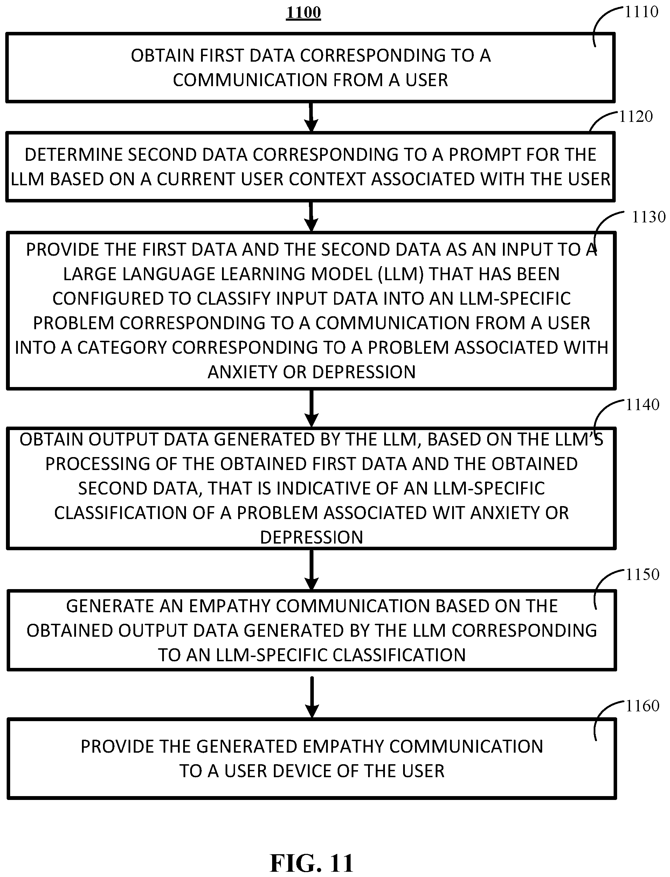

FIG. 11 is a flowchart of another process 1100 for providing empathy to a user based on a classification of a user communication into a problem associated with anxiety or depression, in accordance with one aspect of the present disclosure. For purposes of this disclosure, the process 1100 will be described as being performed by a server such as the server 130 of FIG. 1. However, the process 1100 can also be performed by any computer such as the user device 610 of FIG. 6.

A server can begin execution of the process 1100 by using one or more computers to obtain first data corresponding to a communication from a use (1110).

The server can continue execution of the process 1100 by using one or more computers to determine second data corresponding to a prompt for the LLM based on a current context associated with the user (1120).

The server can continue execution of the process 1100 by using one or more computers to provide (i) the first data and (ii) the second data as an input to a large language learning model (LLM) that has been configured to classify input data into an LLM-specific problem corresponding to a communication from a user into a category corresponding to a problem associated with anxiety or depression (1130). An LLM-specific problem is a characterization, by the LLM, of a problem related to anxiety or depression that a user is experiencing. This characterization can be, e.g., natural language output of the LLM and is not pigeon-holed into any predetermined classification of problems related to anxiety or depression used by, e.g., the rules-engine of the present disclosure.

The server can continue execution of the process 1100 by using one or more computers to obtain output data generated by the LLM, based on the LLM's processing of the provided data, that is indicative of an LLM-specific classification of a problem associated with anxiety or depression (1140).

The server can continue execution of the process 1100 by using one or more computers to generate an empathy communication based on the obtained output data generated by the LLM corresponding to an LLM-specific classification (1150). In some implementations, the empathy communication can include a communication that communicates the LLM-specific problem to the user of the user device 110. In some implementations, this generated communication can include, for example, a natural language communication to the user that summarizes the problem associated with anxiety or depression that the user is experiencing as framed by the LLM.

The server can continue execution of the process 100 by using one or more computers to provide the generated empathy communication to a user device of the user (1160).

In some implementations, execution of the process 1100 by the server can include the server using one or more computers to map the LLM-specific classification that indicates a classification of a problem associated with anxiety or depression to one particular problem classification of a plurality of different problem classifications, wherein each problem classification of the plurality of different problem classifications corresponds to a predetermined problem associated with anxiety or depression. In some implementations, the plurality of different predetermined problem classifications associated with anxiety or depression comprises at least one of a relationship problem, procrastination, grief, sleep, financial, a major sickness, a minor sickness, loneliness, addiction, pain, anxiety, depression, anger, guilt, regret, or corona. In some implementations, execution of the process 1100 by the server can include the server using one or more computers to determine a therapeutic treatment for the user based on the particular problem classification.

In some implementations, the LLM has been configured using rule-example pairs.

In some implementations, the rule-example pair comprises a rule that the LLM is configured to follow and an example of the rule the LLM is configured to follow.

In some implementations, the rule-example pair configures the LLM with limits in the communication the LLM can output to the user device for review by the user.

In some implementations, execution of the process 1100 by the server can also provide the current user context as an input to the LLM. In such implementations, execution of the providing stage 1130 by the server can include the server using one or more computers to provide the obtained first data, the obtained second data, and the current user context as inputs to a large language learning model (LLM) that has been configured to generate output data indicating whether a user is in need of an empathy communication based on processing data corresponding to (i) a communication from a user, (ii) a prompt, and (iii) a current user context.

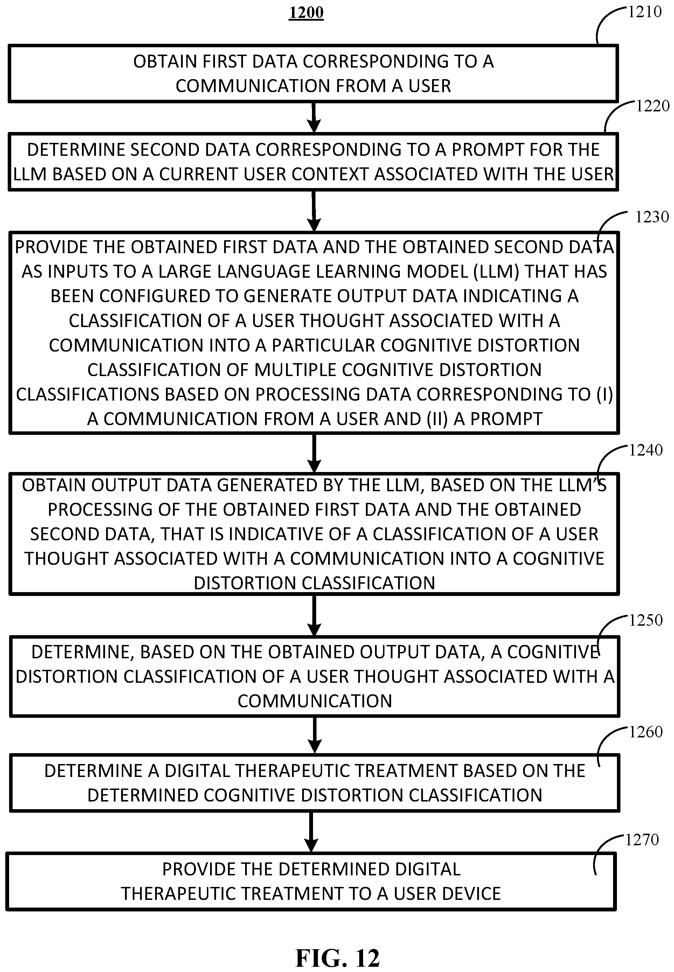

FIG. 12 is a flowchart of a process 1200 for generating a thought challenging treatment for a user, in accordance with one aspect of the present disclosure. For purposes of this disclosure, the process 1200 will be described as being performed by a server such as the server 130 of FIG. 1. However, the process 1200 can also be performed by any computer such as the user device 610 of FIG. 6.

A server can begin execution of the process 1200 by using one or more computers to obtain first data corresponding to a communication from a user (1210).

The server can continue execution of the process 1200 by using one or more computers to determine second data corresponding to a prompt for the LLM based on a current context associated with the user (1220).

The server can continue execution of the process 1200 by using one or more computers to provide the obtained first data and the obtained second data as inputs to a large language learning model (LLM) that has been configured to generate output data indicating a classification of a user thought associated with a communication into a particular cognitive distortion classification of multiple cognitive distortion classifications based on processing data corresponding to (i) a communication from a user and (ii) a prompt (1230).

The server can continue execution of the process 1200 by using one or more computers to obtain output data generated by the LLM, based on the LLM's processing of the obtained first data and the obtained second data, that is indicative of a classification of a user thought associated with a communication into a cognitive distortion classification (1240).

The server can continue execution of the process 1200 by using one or more computers to determine, based on the obtained output data, a cognitive distortion classification of a user thought associated with a communication (1250).

The server can continue execution of the process 1200 by using one or more computers to determine a digital therapeutic treatment based on the determined cognitive distortion classification (1260).

The server can continue execution of the process 1200 by using one or more computes to provide the determined digital therapeutic treatment to a user device (1270).

In some implementations, the multiple cognitive distortion classifications comprise emotional reasoning, labeling, mental filter, overgeneralization, blame, discounting the positive, fortune telling, all or nothing thinking, should statements, and magnification.

In some implementations, the multiple cognitive distortion classifications comprise at least one of emotional reasoning, labeling, mental filter, overgeneralization, blame, discounting the positive, fortune telling, all or nothing thinking, should statements, or magnification.

In some implementations, the determined cognitive classification comprises at least one of emotional reasoning, labeling, mental filter, overgeneralization, blame, discounting the positive, fortune telling, all or nothing thinking, should statements, or magnification.

In some implementations, execution of the process 1200 by the server can also provide the current user context as an input to the LLM. In such implementations, execution of the providing stage 1230 by the server can include the server using one or more computers to provide the obtained first data, the obtained second data, and the current user context as inputs to a large language learning model (LLM that has been configured to generate output data indicating a classification of a user thought associated with a communication into a particular cognitive distortion classification of multiple cognitive distortion classifications based on processing data corresponding to (i) a communication from a user, (ii) a prompt, and (iii) a current user context.

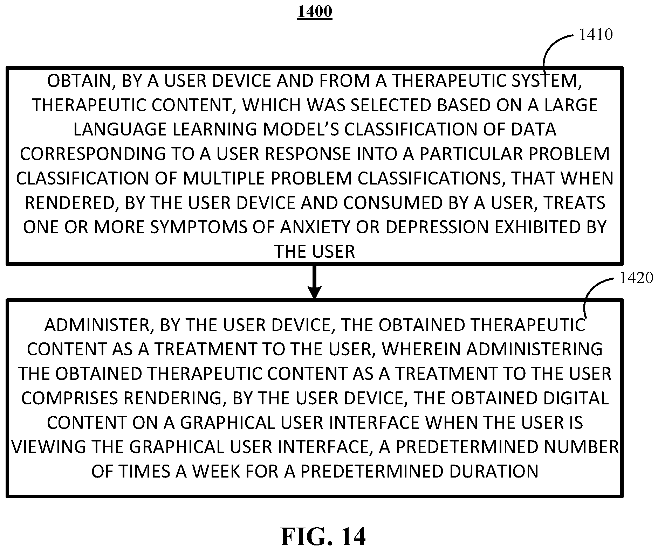

In some implementations, a therapeutic application of a user device is configured to administer the determined digital therapeutic treatment to: receive the provided the provided digital therapeutic that was determined based on the determined cognitive distortion classification, and administer the determined digital therapeutic treatment to the user of the user device. In some implementations, the therapeutic application of the user device can administer the determined digital therapeutic treatment using the same techniques described with respect to the administration of a digital therapeutic as described with reference to FIG. 1 and 14.

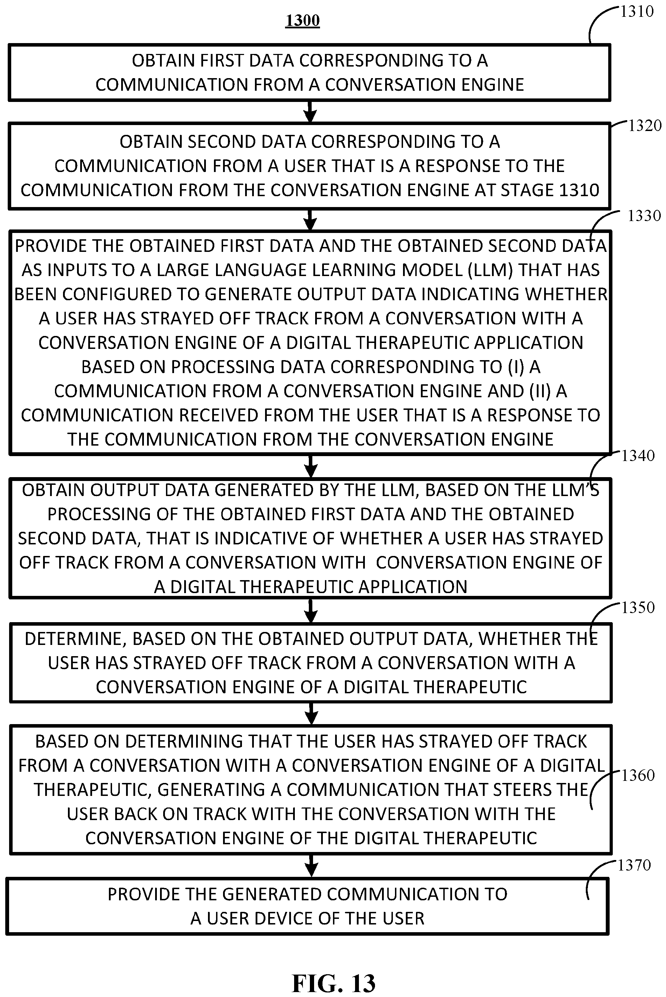

FIG. 13 is a flowchart of a process 1300 for keeping a user on track with a conversation with a conversation engine of a digital therapeutic application, in accordance with one aspect of the present disclosure. For purposes of this disclosure, the process 1300 will be described as being performed by a server such as the server 130 of FIG. 1. However, the process 1300 can also be performed by any computer such as the user device 610 of FIG. 6.

A server can begin execution of the process 1300 by using one or more computers to obtain first data corresponding to a communication from a conversation engine (1310).

The server can continue execution of the process 1300 by using one or more computers to obtain second data corresponding to a communication from a user that is a response to the communication from the conversation engine at stage 1310 (1320). For example, the communication from the conversation engine 131 to the user may be "Hello, how are you doing today?" and a communication in response to that communication from the conversation engine may be "I am doing well, thanks for asking." In this example, the user's response does not stray from the conversation. In another example, a communication from the conversation engine 131 to the user may be "I am looking forward to our conversation today" and a communication in response to that communication from the conversation engine may be "The sky is blue." In this example, the user provided a response to the conversation engine's 131 communication because the user's response came after the conversation engine's communication. However, in this second example, the user's response strays from the conversation because "The sky is blue" strays from the conversation engine's 131 communication of "I look forward to our conversation today."

The server can continue execution of the process 1300 by using one or more computer to provide the obtained first data and the obtained second data as inputs to a large language learning model (LLM) that has been configured to generate output data indicating whether a user has strayed off track from a conversation with a conversation engine of a digital therapeutic application based on processing data corresponding to (i) a first communication from a conversation engine and (ii) a communication received from the user that is a response to the communication from the conversation engine (1330).

The server can continue execution of the process 1300 by using one or more computers to obtain output data generated by the LLM, based on the LLM's processing of the obtained first data and the obtained second data, that is indicative of whether a user has strayed off track from a conversation with a conversation engine of a digital therapeutic application (1340).

The server can continue execution of the process 1300 by using one or more computers to determine, based on the obtained output data, whether the user has strayed off track from a conversation with a conversation engine of a digital therapeutic application (1350).

Based on determining, by one or more computers of the server, that the user has strayed off track from a conversation with a conversation engine of a digital therapeutic, the server can continue execution of the process 1300 by using one or more computers to generate a communication that steers the user back on track with the conversation with the conversation engine of the digital therapeutic (1360). Alternatively, if the server determines that the user has not strayed off track from a conversation with a conversation engine of a digital therapeutic, the process 1300 may terminate. In such instances, the process 1300 can be executed again, beginning at stage 1310, when another communication from the user is received.

The server can continue execution of the process 1300 by using one or more computers to provide the generated communication to a user device of the user (1370).

In some implementations, the LLM has been configured using rule-example pairs.

In some implementations, the rule-example pair comprises a rule that the LLM is configured to follow and an example of the rule the LLM is configured to follow.

In some implementations, the rule-example pair configures the LLM with limits in the communication the LLM can output to the user device for review by the user.

In some implementations, the method can further include determining, by one or more computers, a prompt that is to be provided to the LLM based on the determined user context; and wherein providing, by one or more computers, the obtained first data and the obtained second data as inputs to a large language learning model (LLM) that has been configured to generate output data indicating whether a user has strayed off track from a conversation with a conversation engine of a digital therapeutic application based on processing data corresponding to (i) a first communication from a conversation engine and (ii) a communication received from the user that is a response to the communication from the conversation engine further comprises: providing, by one or more computers, the obtained first data, the obtained second data, and the determined prompt as inputs to a large language learning model (LLM) that has been configured to generate output data indicating whether a user has strayed off track from a conversation with a conversation engine of a digital therapeutic application based on processing data corresponding to (i) a first communication from a conversation engine, (ii) a communication received from the user that is a response to the communication from the conversation engine, and (iii) the determined prompt.

In some implementations, the method can further include determining, by one or more computers, a prompt that is to be provided to the LLM based on the determined user context; and wherein providing, by one or more computers, the obtained first data and the obtained second data as inputs to a large language learning model (LLM) that has been configured to generate output data indicating whether a user has strayed off track from a conversation with a conversation engine of a digital therapeutic application based on processing data corresponding to (i) a first communication from a conversation engine and (ii) a communication received from the user that is a response to the communication from the conversation engine further comprises: providing, by one or more computers, the obtained first data, the obtained second data, the determined prompt, and the current user context as inputs to a large language learning model (LLM) that has been configured to generate output data indicating whether a user has strayed off track from a conversation with a conversation engine of a digital therapeutic application based on processing data corresponding to (i) a first communication from a conversation engine, (ii) a communication received from the user that is a response to the communication from the conversation engine, (iii) the determined prompt, and (iv) a current user context. FIG. 14 is a flowchart of a process 1400 for administering a digital therapeutic selected based on the output of a large language model to a user, in accordance with one aspect of the present disclosure. For purposes of this disclosure, the process 1400 will be described as being performed by a user device such as the user device 110 of FIG.

A user device can being execution of the process 1400 by obtaining, from a therapeutic system, therapeutic content, which was selected based on a large language learning model's classification of data corresponding to a user response into a particular problem classification of multiple problem classifications, that when rendered, by the user device and consumed by a user, treats one or more symptoms of anxiety or depression exhibited by the user (1410).

The user device can continue execution of the process 1400 by administering the obtained therapeutic content as a treatment to the user, wherein administering the obtained therapeutic content as a treatment to the user comprises rendering, by the user device, the obtained digital content on a graphical user interface when the user is viewing the graphical user interface, a predetermined number of times a week for a predetermined duration (1420).

In some implementations, the problem classifications can include at least one of a relationship problem, procrastination, grief, sleep, financial, a major sickness, a minor sickness, loneliness, addiction, pain, anxiety, depression, anger, guilt, regret, or corona.

In some implementations, the large language model (LLM) has been configured using rule-example pairs.

In some implementations, the rule-example pair comprises a rule that the LLM is configured to follow and an example of the rule the LLM is configured to follow.

In some implementations, the rule-example pair configures the LLM with limits in the communication the LLM can output to the user device for review by the user.

In some implementations, the obtained therapeutic content comprises one or more interpersonal psychotherapy (IPT) tools or one or more cognitive behavioral therapy (CBT) tools.

In some implementations, the obtained therapeutic content comprises one or more interpersonal psychotherapy (IPT) tools and one or more cognitive behavioral therapy (CBT) tools. In such implementations, the user device can administer the obtained therapeutic content as a treatment to the user by rendering, the one or more interpersonal psychotherapy (IPT) tools and one or more cognitive behavioral therapy (CBT) tools in the graphical user interface of the user device a predetermined number of times a week for a duration of two to eight weeks.

In some implementations, the obtained therapeutic content comprises one or more interactive cognitive behavioral therapy (CBT) tools. In the user device can administer the obtained therapeutic content as a treatment to the user by using the user device to deliver the one or more interactive cognitive behavioral therapy (CBT) tools that comprises a natural language interaction with the user.

In some implementations, the natural language interaction with the user is between a user and the large language model. In some implementations, the natural language interaction can include (i) one or more natural language communications from the large language model to the user of the user device and (ii) one or more natural language responses from the user of the user device to the large language model. In some implementations, the natural language interaction with the user is between the user and a conversational engine that is different from the large language model. In some implementations, the natural language interaction can include (i) one or more natural language communications from the conversational engine to the user of the user device and (ii) one or more natural language responses from the user of the user device to the conversational engine.

In some implementations, the predetermined duration is two to eight weeks.

In some implementations, the predetermined duration is at least 8 weeks.

In some implementations, the therapeutic content is administered for at least 5 minutes during each of the predetermined number of times per week.

In some implementations, the obtained therapeutic content is administered at least 10 minutes during each of the predetermined number of times per week.

In some implementations, the predetermined number of times a week for a predetermined duration comprises at least 4 weeks of an 8-week time period.

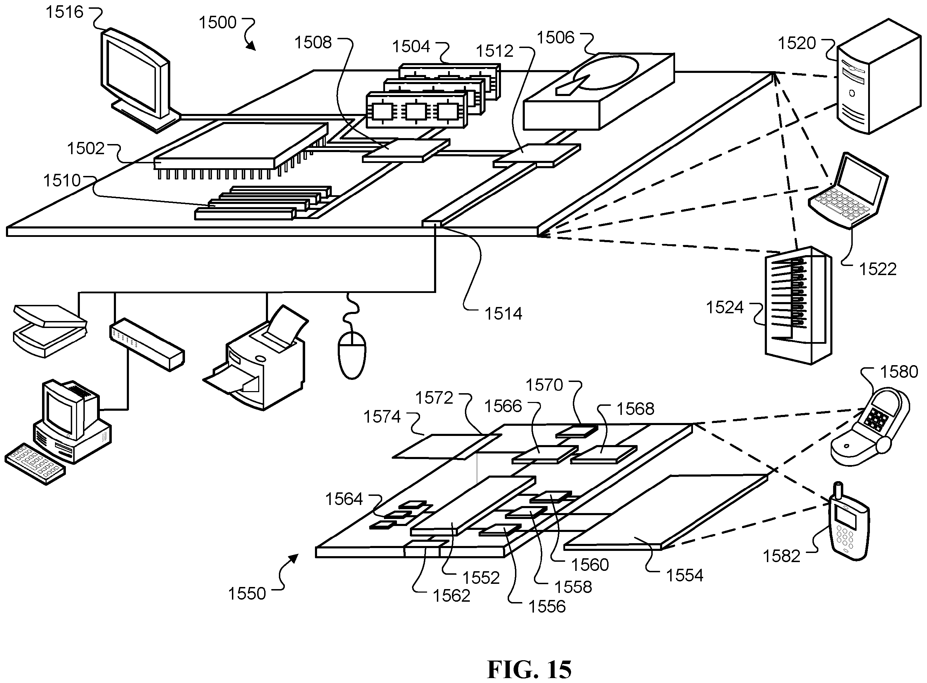

FIG. 15 is a block diagram of system components that can be used to implement a system for generating, dosing, and administering a digital therapeutic to one or more symptoms related to a mental health condition.

Computing device 1500 is intended to represent various forms of digital computers, such as laptops, desktops, workstations, personal digital assistants, servers, blade servers, mainframes, and other appropriate computers. Computing device 1550 is intended to represent various forms of mobile devices, such as personal digital assistants, cellular telephones, smartphones, and other similar computing devices. Additionally, computing device 1500 or 1550 can include Universal Serial Bus (USB) flash drives. The USB flash drives can store operating systems and other applications. The USB flash drives can include input/output components, such as a wireless transmitter or USB connector that can be inserted into a USB port of another computing device. The components shown here, their connections and relationships, and their functions, are meant to be exemplary only, and are not meant to limit implementations of the inventions described and/or claimed in this document.

Computing device 1500 includes a processor 1502, memory 1504, a storage device 1506, a high-speed interface 1508 connecting to memory 1504 and high-speed expansion ports 1510, and a low-speed interface 1512 connecting to low-speed bus 1514 and storage device 1506. Each of the components 1502, 1504, 1506, 1508, 1510, and 1512, are interconnected using various busses, and can be mounted on a common motherboard or in other manners as appropriate. The processor 1502 can process instructions for execution within the computing device 1500, including instructions stored in the memory 1504 or on the storage device 1506 to display graphical information for a GUI on an external input/output device, such as display 1516 coupled to high-speed interface 1508. In other implementations, multiple processors and/or multiple buses can be used, as appropriate, along with multiple memories and types of memory. Also, multiple computing devices 1500 can be connected, with each device providing portions of the necessary operations, e.g., as a server bank, a group of blade servers, or a multi-processor system.

The memory 1504 stores information within the computing device 1500. In one implementation, the memory 1504 is a volatile memory unit or units. In another implementation, the memory 1504 is a non-volatile memory unit or units. The memory 1504 can also be another form of computer-readable medium, such as a magnetic or optical disk.

The storage device 1506 is capable of providing mass storage for the computing device 1500. In one implementation, the storage device 1506 can be or contain a computer-readable medium, such as a floppy disk device, a hard disk device, an optical disk device, or a tape device, a flash memory or other similar solid state memory device, or an array of devices, including devices in a storage area network or other configurations. A computer program product can be tangibly embodied in an information carrier. The computer program product can also contain instructions that, when executed, perform one or more methods, such as those described above. The information carrier is a computer- or machine-readable medium, such as the memory 1504, the storage device 1506, or memory on processor 1502.

The high-speed controller 1508 manages bandwidth-intensive operations for the computing device 1500, while the low-speed controller 1512 manages lower bandwidth intensive operations. Such allocation of functions is exemplary only. In one implementation, the high-speed controller 1508 is coupled to memory 1504, display 1516, e.g., through a graphics processor or accelerator, and to high-speed expansion ports 1510, which can accept various expansion cards (not shown). In the implementation, low-speed controller 1512 is coupled to storage device 1506 and low-speed expansion port 1514. The low-speed expansion port, which can include various communication ports, e.g., USB, Bluetooth, Ethernet, wireless Ethernet can be coupled to one or more input/output devices, such as a keyboard, a pointing device, microphone/speaker pair, a scanner, or a networking device such as a switch or router, e.g., through a network adapter. The computing device 1500 can be implemented in a number of different forms, as shown in the figure. For example, it can be implemented as a standard server 1520, or multiple times in a group of such servers. It can also be implemented as part of a rack server system 1524. In addition, it can be implemented in a personal computer such as a laptop computer 1522. Alternatively, components from computing device 1500 can be combined with other components in a mobile device (not shown), such as device 1550. Each of such devices can contain one or more of computing device 1500, 1550, and an entire system can be made up of multiple computing devices 1500, 1550 communicating with each other.

The computing device 1500 can be implemented in a number of different forms, as shown in the figure. For example, it can be implemented as a standard server 1520, or multiple times in a group of such servers. It can also be implemented as part of a rack server system 1524. In addition, it can be implemented in a personal computer such as a laptop computer 1522. Alternatively, components from computing device 1500 can be combined with other components in a mobile device (not shown), such as device 1550. Each of such devices can contain one or more of computing device 1500, 1550, and an entire system can be made up of multiple computing devices 1500, 1550 communicating with each other

Computing device 1550 includes a processor 1552, memory 1564, and an input/output device such as a display 1554, a communication interface 1566, and a transceiver 1568, among other components. The device 1550 can also be provided with a storage device, such as a micro-drive or other device, to provide additional storage. Each of the components 1550, 1552, 1564, 1554, 1566, and 1568, are interconnected using various buses, and several of the components can be mounted on a common motherboard or in other manners as appropriate.

The processor 1552 can execute instructions within the computing device 1550, including instructions stored in the memory 1564. The processor can be implemented as a chipset of chips that include separate and multiple analog and digital processors. Additionally, the processor can be implemented using any of a number of architectures. For example, the processor 1510 can be a CISC (Complex Instruction Set Computers) processor, a RISC (Reduced Instruction Set Computer) processor, or a MISC (Minimal Instruction Set Computer) processor. The processor can provide, for example, for coordination of the other components of the device 1550, such as control of user interfaces, applications run by device 1550, and wireless communication by device 1550.

Processor 1552 can communicate with a user through control interface 1558 and display interface 1556 coupled to a display 1554. The display 1554 can be, for example, a TFT (Thin-Film-Transistor Liquid Crystal Display) display or an OLED (Organic Light Emitting Diode) display, or other appropriate display technology. The display interface 1556 can comprise appropriate circuitry for driving the display 1554 to present graphical and other information to a user. The control interface 1558 can receive commands from a user and convert them for submission to the processor 1552. In addition, an external interface 1562 can be provide in communication with processor 1552, so as to enable near area communication of device 1550 with other devices. External interface 1562 can provide, for example, for wired communication in some implementations, or for wireless communication in other implementations, and multiple interfaces can also be used.

The memory 1564 stores information within the computing device 1550. The memory 1564 can be implemented as one or more of a computer-readable medium or media, a volatile memory unit or units, or a non-volatile memory unit or units. Expansion memory 1574 can also be provided and connected to device 1550 through expansion interface 1572, which can include, for example, a SIMM (Single In Line Memory Module) card interface. Such expansion memory 1574 can provide extra storage space for device 1550, or can also store applications or other information for device 1550. Specifically, expansion memory 1574 can include instructions to carry out or supplement the processes described above, and can include secure information also. Thus, for example, expansion memory 1574 can be provide as a security module for device 1550, and can be programmed with instructions that permit secure use of device 1550. In addition, secure applications can be provided via the SIMM cards, along with additional information, such as placing identifying information on the SIMM card in a non-hackable manner.

The memory can include, for example, flash memory and/or NVRAM memory, as discussed below. In one implementation, a computer program product is tangibly embodied in an information carrier. The computer program product contains instructions that, when executed, perform one or more methods, such as those described above. The information carrier is a computer- or machine-readable medium, such as the memory 1564, expansion memory 1574, or memory on processor 1552 that can be received, for example, over transceiver 1568 or external interface 1562.

Device 1550 can communicate wirelessly through communication interface 1566, which can include digital signal processing circuitry where necessary. Communication interface 1566 can provide for communications under various modes or protocols, such as GSM voice calls, SMS, EMS, or MMS messaging, CDMA, TDMA, PDC, WCDMA, CDMA2000, or GPRS, among others. Such communication can occur, for example, through radio-frequency transceiver 1568. In addition, short-range communication can occur, such as using a Bluetooth, Wi-Fi, or other such transceiver (not shown). In addition, GPS (Global Positioning System) receiver module 1570 can provide additional navigation- and location-related wireless data to device 1550, which can be used as appropriate by applications running on device 1550.

Device 1550 can also communicate audibly using audio codec 1560, which can receive spoken information from a user and convert it to usable digital information. Audio codec 1560 can likewise generate audible sound for a user, such as through a speaker, e.g., in a handset of device 1550. Such sound can include sound from voice telephone calls, can include recorded sound, e.g., voice messages, music files, etc. and can also include sound generated by applications operating on device 1550.

The computing device 1550 can be implemented in a number of different forms, as shown in the figure. For example, it can be implemented as a cellular telephone 1580. It can also be implemented as part of a smartphone 1582, personal digital assistant, or other similar mobile device.

Various implementations of the systems and methods described here can be realized in digital electronic circuitry, integrated circuitry, specially designed ASICs (application specific integrated circuits), computer hardware, firmware, software, and/or combinations of such implementations. These various implementations can include implementation in one or more computer programs that are executable and/or interpretable on a programmable system including at least one programmable processor, which can be special or general purpose, coupled to receive data and instructions from, and to transmit data and instructions to, a storage system, at least one input device, and at least one output device.

These computer programs (also known as programs, software, software applications or code) include machine instructions for a programmable processor, and can be implemented in a high-level procedural and/or object-oriented programming language, and/or in assembly/machine language. As used herein, the terms "machine-readable medium" "computer-readable medium" refers to any computer program product, apparatus and/or device, e.g., magnetic discs, optical disks, memory, Programmable Logic Devices (PLDs), used to provide machine instructions and/or data to a programmable processor, including a machine-readable medium that receives machine instructions as a machine-readable signal. The term "machine-readable signal" refers to any signal used to provide machine instructions and/or data to a programmable processor.

To provide for interaction with a user, the systems and techniques described here can be implemented on a computer having a display device, e.g., a CRT (cathode ray tube) or LCD (liquid crystal display) monitor for displaying information to the user and a keyboard and a pointing device, e.g., a mouse or a trackball by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input.

The systems and techniques described here can be implemented in a computing system that includes a back end component, e.g., as a data server, or that includes a middleware component, e.g., an application server, or that includes a front end component, e.g., a client computer having a graphical user interface or a Web browser through which a user can interact with an implementation of the systems and techniques described here, or any combination of such back end, middleware, or front end components. The components of the system can be interconnected by any form or medium of digital data communication, e.g., a communication network. Examples of communication networks include a local area network ("LAN"), a wide area network ("WAN"), and the Internet.

The computing system can include clients and servers. A client and server are generally remote from each other and typically interact through a communication network. The relationship of client and server arises by virtue of computer programs running on the respective computers and having a client-server relationship to each other.

Embodiments can be implemented in digital electronic circuitry, or in computer hardware, firmware, software, or in combinations thereof. Apparatus of the invention can be implemented in a computer program product tangibly embodied or stored in a machine-readable storage device for execution by a programmable processor; and method actions can be performed by a programmable processor executing a program of instructions to perform functions of the invention by operating on input data and generating output. The invention can be implemented advantageously in one or more computer programs that are executable on a programmable system including at least one programmable processor coupled to receive data and instructions from, and to transmit data and instructions to, a data storage system, at least one input device, and at least one output device. Each computer program can be implemented in a high-level procedural or object-oriented programming language, or in assembly or machine language if desired; and in any case, the language can be a compiled or interpreted language.

Suitable processors include, by way of example, both general and special purpose microprocessors. Generally, a processor will receive instructions and data from a read-only memory and/or a random-access memory. Generally, a computer will include one or more mass storage devices for storing data files; such devices include magnetic disks, such as internal hard disks and removable disks; magneto-optical disks; and optical disks. Storage devices suitable for tangibly embodying computer program instructions and data include all forms of non-volatile memory, including by way of example semiconductor memory devices, such as EPROM, EEPROM, and flash memory devices; magnetic disks such as internal hard disks and removable disks; magneto-optical disks; and CD_ROM disks. Any of the foregoing can be supplemented by, or incorporated in, ASICs (application-specific integrated circuits).

Other embodiments are within the scope and spirit of the description claims. Due to the nature of software, functions described above can be implemented using software, hardware, firmware, hardwiring, or combinations of any of these. Features implementing functions may also be physically located at various positions, including being distributed such that portions of functions are implemented at different physical locations.

### EXAMPLE 1

### Anxiety or Depression Using Rules-Based Conversation Engine

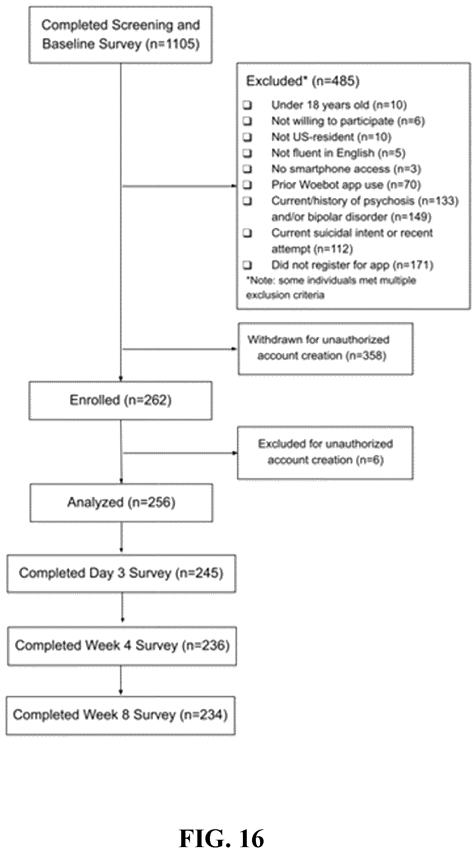

FIG. 16 is a CONSORT diagram describing participant selection and participate in a study that used the present disclosure to treat anxiety or depression. As detailed in the CONSORT of FIG. 16, 1105 potential participants were screened. Of these, 485 were excluded for not meeting eligibility criteria. After enrollment, an additional 358 participants with unauthorized accounts (e.g., duplicate registrants) were withdrawn from the study and excluded from the analyses. An additional six unauthorized registrants completed study procedures and were subsequently removed from the analytic sample, given that they were initially missed as a result of a clerical error. Thus, the final sample included in analyses consisted of 256 participants. Of these 256 participants, 245 completed the day 3 survey, 236 completed the week 4 surveys, and 234 completed the week 9 surveys.

Although Day 3 survey data were collected as part of the parent study, they were not part of the analyses included in the present report.

### Sample Characteristics

The analytic subsamples included 111 participants with elevated baseline levels of depressive symptoms and 1 07 participants with elevated baseline levels of anxiety symptoms at baseline as defined by a PHQ-8 score ≥ 10 and/or GAD-7 score ≥ 10, respectively. The sample size for the combined (elevated on PHQ-8 or GAD-7) groups comprised 139 participants. Some participants with elevated symptoms of both depressive and anxiety symptoms were in both analytic subsamples (n=79). Both the depressive symptom and anxiety symptom samples were predominantly female, educated, and employed, identified as non-Hispanic white and heterosexual, and had private health insurance (Table 1 of Example 1) with mean ages of 38 and 37 years, respectively. Among those with clinical levels of depressive symptoms, 50% had symptoms falling in the moderately severe or severe range (PHQ-8 score ≥ 15) and 49% had concurrent mental health treatment during the study. Among those with clinical levels of anxiety symptoms, 44% had symptoms falling in the severe range (GAD-7 score ≥ 15) and 53% had concurrent mental health treatment during the study.

The assessment completion rate at Week 9 was 91%. The PP population examined in sensitivity analyses comprised 177 (69%) of the 256 participants in the analytic sample after removing 67 (26%) who used WB-LIFE for at most 3 of 9 weeks and removing 22 (9%) whose end of study (EOS) PHQ-8 and GAD-7 assessments at Week 9 were fully missing (no item scores were completed for each measure); the removals were not mutually exclusive. Sixty-four percent (N=71/111) of the elevated depressive symptoms subgroup met per protocol criteria and 59% (N=63/107) of the elevated anxiety symptoms subgroup met these criteria.

**Table 1 of Example 1. Baseline sociodemographic and clinical characteristics, adherence, and satisfaction among participants with clinical levels of baseline depressive or anxiety symptoms.**

| | | Participants with PHQ-8 baseline score ≥ 10 N= 111 n (%) | Participants with GAD-7 baseline score ≥ 10 N= 107 n (%) |
|---|---|---|---|
| **Sociodemographic Characteristic** | | | |
| Age in years (mean, sd) | | 38 (13.57) | 37 (12.28) |
| Race/ethnicity | | | |
| | Non-Hispanic Black | 37 (33%) | 32 (30%) |
| | Non-Hispanic White | 55 (50%) | 56 (52%) |
| | Other | 19 (17%) | 19 (18%) |
| Sex at Birth | | | |
| | Female | 83 (75%) | 81 (76%) |
| | Male | 28 (25%) | 26 (24%) |
| Age in years (mean, sd) | | 38 (13.57) | 37 (12.28) |
| Sexual Orientation | | | |
| | Sexual Minority | 19 (17%) | 19 (18%) |
| | Heterosexual | 92 (83%) | 88 (82%) |
| Education | | | |
| | Graduate or Postgraduate Degree | 32 (29%) | 24 (23%) |
| | College Degree | 43 (39%) | 43 (41%) |
| | Some College or technical school | 21 (19%) | 22 (21%) |
| | High School (Grades 9-15) | 14 (13%) | 16 (15%) |
| Employment | | | |
| | Full Time Employed | 53 (49%) | 57 (54%) |
| | Part Time Employed | 11 (10%) | 9 (9%) |
| | Not Employed | 29 (27%) | 22 (21%) |
| | Other | 15 (14%) | 17 (16%) |
| Marital Status | | | |
| | Divorced/Separated/Widowed | 10 (9%) | 10 (10%) |
| | Married/Partnered/Cohabitating | 50 (47%) | 48 (46%) |
| | Single | 47 (44%) | 47 (45%) |
| Health Insurance | | | |
| | Government Based Insurance | 40 (37%) | 37 (36%) |
| | Private Insurance | 46 (43%) | 45 (43%) |
| | No Insurance/ Prefer not to answer | 21 (20%) | 22 (21%) |
| Age in years (mean, sd) | | 38 (13.57) | 37 (12.28) |

| **Clinical Characteristics** | | | |
|---|---|---|---|
| Baseline Depressive symptom | | | |
| severity | | 0 (0%) | 7 (7%) |
| | Minimal | 0 (0%) | 21 (20%) |
| | Mild | 55 (50%) | 25 (23%) |
| | Moderate | 30 (27%) | 29 (27%) |
| | Moderate-severe | 26 (23%) | 25 (23%) |
| | Severe | | |
| Baseline Anxiety symptom severity | | | |
| | Minimal | 3 (3%) | 0 (0%) |
| | Mild | 29 (26%) | 0 (0%) |
| | Moderate | 37 (33%) | 60 (56%) |
| | Severe | 42 (38%) | 47 (44%) |
| Concurrent Mental Health | | | |
| Treatment* | | 54 (49%) | 57 (53%) |
| | Any (Concurrent treatment) | 57 (51%) | 50 (47%) |
| | None (Woebot only) | | |

| **Adherence Metrics** | | | |
|---|---|---|---|
| App Utilization | | | |
| | | 74 (67%) | 69 (64%) |

| | | Participants with PHQ-8 baseline score ≥ 10 N= 111 n (%) | Participants with GAD-7 baseline score ≥ 10 N= 107 n (%) |
|---|---|---|---|
| **Sociodemographic Characteristic** | | | |
| Age in years (mean, sd) | | 38 (13.57) | 37 (12.28) |
| Use of WB-LIFE in at least 4 of 9 weeks | | | |
| Per protocol data set | | 71 (64%) | 63 (59%) |

GAD-7 = Generalized Anxiety DIsorder-7 item scale; PHQ-8 = Patient Health Questionnaire-8 item scale
* Concurrent mental health treatment = any psychotherapy or psychotropic medication use at any time during the study
"Per protocol" was defined as using WB-LIFE in at least 4 of 9 study weeks and completing end of study PHQ-8 and GAD-7 assessments.

### Depressive Symptoms

In the subgroup with clinically elevated depressive symptoms, PHQ-8 scores significantly decreased over the 9-week study period (mean change -7.28, p < .001). In bivariate regression models, the amount of symptom decline did not significantly differ by age, educational level, employment, or baseline level of anxiety symptoms but did significantly differ across several characteristics tested (Table 2 of Example 1). Non-Hispanic Blacks, those who were single, and those with severe levels of baseline depressive symptoms demonstrated significantly greater amounts of decline in depressive symptoms than Non-Hispanic Whites, those who were married/partnered/cohabitating, and those with moderate levels of baseline depressive symptoms, respectively. Females, participants identifying as sexual minorities, those with private or government-based health insurance, and those in concurrent mental health treatment demonstrated significantly lower amounts of decline in depressive symptoms than males, participants identifying as heterosexuals, those with no health insurance or who declined to answer the health insurance question, and those not in concurrent mental health treatment, respectively. Per protocol analyses largely confirmed the ITT findings (Supplementary Table 1 of Example 1).

After removing race/ethnicity, insurance, and employment from consideration because of multicollinearity, the final multiple regression model indicated significant differences in the amount of decrease in depressive symptoms at Week 9 by sexual orientation, marital status, baseline depressive symptom severity category, and concurrent mental health treatment (Table 3 of Example 1) in the same directions as each bivariate model described above.

**Table 2 of Example 1. Unadjusted Bivariate Linear Regression Models of Characteristics Associated with change scores from baseline to Week 9 in depressive symptoms among those with clinically elevated levels of baseline depressive symptoms: PHQ-8 ≥ 10.**

| | | **PHQ-8: Week 9 Change Scores** | | |
|---|---|---|---|---|
| *Characteristics of Interest* | | *Estimates* | *95% CI* | *p-value* |
| Use of WB-LIFE in at least 4 of 9 weeks | | 2.9 | 0.51, 5.4 | **0.018** |
| Age | | 0.03 | -0.05, 0.12 | 0.4 |
| Race/Ethnicity | | Reference Level | | |
| | Non-Hispanic White | | | |
| | Non-Hispanic Black | -6.0 | -8.3, -3.7 | **<0.001** |
| | Other | 0.02 | -2.8, 2.9 | >0.9 |
| Sex at Birth | | | | |
| | Male | Reference Level | | |
| | Female | 2.7 | 0.09, 5.3 | **0.042** |
| Sexual Orientation: | | | | |
| | Heterosexual | Reference Level | | |
| | Sexual Minority | 5.6 | 2.6, 9.6 | **<0.001** |
| Education | | | | |
| | High School | Reference Level | | |
| | College Degree | -1.9 | -5.6, 1.8 | 0.3 |
| | Graduate or postgraduate degree | -0.68 | -4.4, 3.1 | 0.7 |
| | Some college or technical school | 1.1 | -3.1, 5.3 | 0.6 |
| Employment | | Reference Level | | |
| | Full Time | | | |
| | Not Employed | 2.4 | -0.36, 5.2 | 0.087 |
| | Other | 3.1 | -0.49, 6.8 | 0.089 |
| | Part Time Employed | 3.6 | -0.32, 7.4 | 0.071 |
| Marital Status | | | | |
| | Married/Partnered/Cohabiting | Reference Level | | |
| | Divorced/Separated/Widowed | -2.2 | -6.2, 1.7 | 0.3 |
| | Single | -4.1 | -6.6, -1.7 | **<0.001** |
| Health Insurance: | | | | |
| | No Insurance/Prefer not to answer | Reference Level | | |
| | Government based Insurance | 6.9 | 4.2, 9.7 | **<0.001** |
| | Private Insurance | 7.5 | 4.8, 10 | **<0.001** |
| BL Depressive Symptom Severity | | | | |
| | Moderate | Reference Level | | |
| | Moderate-Severe | -1.1 | -3.2, 1.0 | 0.3 |
| | Severe | -9.3 | -12, -7.0 | **<0.001** |
| BL Anxiety Symptom Severity | | | | |
| | Minimal | Reference Level | | |
| | Mild | -1.6 | -9.5, 6.2 | 0.7 |
| | Moderate | -2.1 | -9.9, 5.7 | 0.6 |
| | Severe | -7.1 | -15, 0.71 | 0.074 |
| Concurrent Mental Health Treatment* | | 3.2 | 0.94, 5.4 | **0.006** |

| | | | | |
|---|---|---|---|---|
| BL = baseline; PHQ-8 = Patient Health Questionnaire-8 item scale * Concurrent mental health treatment = any psychotherapy or psychotropic medication use at any time during the study | | | | |

**Table 3 of Example 1. Linear Regression Model (Multiple Regression) of change scores from baseline to Week 9 in depressive symptoms among those with clinically elevated levels of baseline depressive symptoms: PHQ-8 ≥ 10.**

| | | **PHQ-8: Week 9 Change Scores** | | |
|---|---|---|---|---|
| *Predictors* | | *Estimates* | *95% CI* | *p-value* |
| (Intercept) | | -2.46 | -10.16 - 5.24 | 0.527 |
| Used the app in at least 4 of 9 weeks | | 0.2 | -1.75 -2.15 | 0.84 |
| Age | | 0.05 | -0.03 - 0.14 | 0.229 |
| Sex at Birth | | | | |
| | Male | Reference Level | | |
| | Female | -0.96 | -3.24 - 1.32 | 0.405 |
| Sexual Orientation | | | | |
| | Heterosexual | Reference Level | | |
| | Sexual Minority | 5.71 | 2.88 - 9.54 | **<0.001** |
| Education | | Reference Level | | |
| | High School | | | |
| | College Degree | -1.63 | -4.33 - 1.07 | 0.234 |
| | Graduate or postgraduate degree | -0.24 | -3.18 - 2.70 | 0.873 |
| | Some college or technical school | 0.88 | -2.26 - 4.01 | 0.58 |
| Marital Status | | | | |
| | Married/Partnered/Cohabiting | Reference Level | | |
| | Divorced/Separated/Widowed | -3.86 | -7.38 - -0.34 | **0.032** |
| | Single | -3.75 | -5.78 - -1.72 | **<0.001** |
| BL Depressive Symptom Severity | | | | |
| | Moderate | Reference Level | | |
| | Moderate-Severe | -0.09 | -2.44 - 2.25 | 0.937 |
| | Severe | -5.32 | -8.16 - -2.47 | **<0.001** |
| BL Anxiety Symptom Severity | | Reference Level | | |
| | Minimal | | | |
| | Mild | -3.97 | -10.29 - 2.35 | 0.215 |
| | Moderate | -4.5 | -11.02 - 2.02 | 0.173 |
| | Severe | -5.59 | -12.17 - 0.99 | 0.095 |
| Concurrent Mental Health Treatment* | | 2.62 | 0.80 - 4.43 | **0.005** |

| | | | | |
|---|---|---|---|---|
| BL = baseline; PHQ-8 = Patient Health Questionnaire-8 item scale * Concurrent mental health treatment = any psychotherapy or psychotropic medication use at any time during the study NOTE: Final models did not consider race/ethnicity, employment, or insurance status because of multicollinearity. | | | | |

### Anxiety Symptoms

Average GAD-7 scores for each subgroup of interest significantly improved over the course of the study. In the subgroup with clinically elevated anxiety symptoms at baseline, GAD-7 scores significantly decreased over the 9-week study period (mean change -7.45, p < .001). The amount of symptom decline did not significantly differ by use of WB-LIFE at least 4 of 9 weeks, age, sex at birth, educational level, or employment, but did significantly differ across several characteristics tested (Table 4 of Example 1). Non-Hispanic Blacks, those who were single, those with severe levels of baseline depressive symptoms, and those with severe levels of anxiety symptoms demonstrated greater amounts of decline in anxiety symptoms than Non-Hispanic Whites, those who were married/partnered/cohabitating, those with moderate levels of baseline depressive symptoms, and those with moderate levels of baseline anxiety symptoms. Participants identifying as sexual minorities, those with private or government-based health insurance, and those in concurrent mental health treatment demonstrated lower amounts of decline in anxiety symptoms than heterosexuals, those with no health insurance or who declined to answer the health insurance question, and those without concurrent mental health treatment, respectively. Per protocol analyses largely confirmed the full sample findings, with the exception of concurrent mental health treatment reaching marginal significance (p = .052, Supplementary Table 2 of Example 1).

After removing race/ethnicity, employment, and insurance status from consideration due to multicollinearity, the final multiple regression model indicated significant differences in the amount of decrease in anxiety symptoms at Week 9 by sexual orientation, marital status, baseline anxiety symptom severity category, and concurrent mental health treatment (Table 5 of Example 1) in the same direction as those previously described in each bivariate regression model.

**Table 4 of Example 1: Unadjusted Bivariate Linear Regression Models of change scores from baseline to Week 9 in anxiety symptoms among those with clinically elevated levels of baseline anxiety symptoms: GAD-7 ≥ 10**

| | | **GAD-7: Week 9 Change Scores** | | |
|---|---|---|---|---|
| *Characteristics of Interest* | | *Estimates* | *95% CI* | *p-value* |
| Use of WB-LIFE in at least 4 of 9 weeks | | 2.2 | -0.25, 4.7 | 0.077 |
| Age | | 0.03 | -0.07, 0.14 | 0.5 |
| Race/Ethnicity | | | | |
| | Non-Hispanic White | Reference Level | | |
| | Non-Hispanic Black | -5.7 | -08.2, -3.2 | **<0.001** |
| | Other | -0.12 | **-3.0, 2.8** | >0.9 |
| Sex at Birth | | | | |
| | Male | Reference Level | | |
| | Female | 1.8 | -1.0, 4.6 | 0.2 |
| Sexual Orientation | | | | |
| | Heterosexual | Reference Level | | |
| | Sexual Minority | 3.7 | 0.65, 6.8 | **0.018** |
| Education | | | | |
| | High School | Reference Level | | |
| | College Degree | -1.7 | -5.3, 1.9 | 0.3 |
| | Graduate or postgraduate degree | -1.8 | -5.7, 2.1 | 0.4 |
| | Some college or technical school | -0.28 | -4.5, 3.9 | 0.9 |
| Employment | | | | |
| | Full Time | Reference Level | | |
| | Not Employed | 2.1 | -1.0, 5.2 | 0.2 |
| | Other | 1.2 | -2.2, 4.7 | 0.5 |
| | Part Time Employed | 2.3 | -2.2, 6.8 | 0.3 |
| Marital Status | | | | |
| | Married/Partnered/Cohabiting | Reference Level | | |
| | Divorced/Separated/Widowed | -1.5 | -5.8, 2.8 | 0.5 |
| | Single | -2.9 | -5.4, -0.37 | **0.025** |
| Health Insurance | | | | |
| | No Insurance/Prefer not to answer | Reference Level | | |
| | Government based Insurance | 5.1 | 2.1, 9.0 | **<0.001** |
| | Private Insurance | 6.8 | 3.9, 9.6 | **<0.001** |
| BL Depressive Symptom Severity | | | | |
| | Minimal | Reference Level | | |
| | Mild | -1.4 | -6.2, 3.4 | 0.6 |
| | Moderate | -0.71 | -5.3, 3.9 | 0.8 |
| | Moderate-Severe | -0.12 | -4.6, 4.4 | >0.9 |
| | Severe | -7.3 | -12, -2.7 | **0.002** |
| BL Anxiety Symptom Severity | | | | |
| | Moderate | Reference Level | | |
| | Severe | -4.9 | -7.1, -2.7 | **<0.001** |
| Concurrent Mental Health Treatment* | | 3.2 | 0.90, 5.6 | **0.007** |

| | | | | |
|---|---|---|---|---|
| BL = baseline; GAD-7 = Generalized Anxiety DIsorder-7 item scale * Concurrent mental health treatment = any psychotherapy or psychotropic medication use at any time during the study. | | | | |

**Table 5 of Example 1. Linear Regression Model (Multiple Regression) of change scores from baseline to Week 9 in anxiety symptoms among those with clinically elevated levels of baseline anxiety symptoms: GAD-7 ≥ 10**

| | | **GAD-7: Week 9 Change Scores** | | |
|---|---|---|---|---|
| *Predictors* | | *Estimates* | *95% CI* | *p-value* |
| (Intercept) | | -4.68 | -10.40 - 1.03 | 0.107 |
| Used the app in at least 4 of 9 weeks | | 1.26 | -1.06 - 3.59 | 0.282 |
| Age | | 0 | -0.11-0.11 | 0.975 |
| Sex at Birth: | | | | |
| | Male | Reference Level | | |
| | Female | -1.76 | -4.63 - 1.10 | 0.224 |
| Sexual Orientation: | | | | |
| | Heterosexual | Reference Level | | |
| | Sexual Minority | 4.47 | 1.20-7.74 | **0.008** |
| Education Level: | | Reference Level | | |
| | High School | | | |
| | College Degree | -1.7 | -4.79 - 1.39 | 0.278 |
| | Graduate or postgraduate degree | -1.12 | -4.72 - 2.48 | 0.536 |
| | Some college or technical school | -0.55 | -4.38 - 3.28 | 0.776 |
| Marital Status: | | | | |
| Married/Partnered/Cohabiting | | Reference Level | | |
| | Divorced/Separated/Widowed | -2.85 | -7.30 - 1.61 | 0.208 |
| | Single | -4.19 | -6.69 - -1.68 | **0.001** |
| BL Anxiety Symptom Severity: | | | | |
| | Moderate | Reference Level | | |
| | Severe | -3.88 | -6.14 - -1.62 | **0.001** |
| Concurrent Mental Health Treatment* | | 3.2 | 0.95 - 5.45 | **0.006** |

| | | | | |
|---|---|---|---|---|
| BL = baseline; GAD-7 = Generalized Anxiety DIsorder-7 item scale * Concurrent mental health treatment = any psychotherapy or psychotropic medication use at any time during the study NOTE: Final models did not consider race/ethnicity, employment, and insurance status due to multicollinearity. | | | | |

### Safety

No study participant reported an AE or serious adverse event (SAE) during the study. Of 256 participants, 151 free text inputs from 71 participants triggered WB-LIFE's potential concerning language detection algorithm. Of these, four participants confirmed being in a crisis situation (resulting in four total confirmations) and were automatically provided with resources and support as described in the Safety section of the Methods.

### Supplemental Table 1 of Example 1. Linear Regression Model (Univariate Regressions) of change scores from baseline to Week 9 in depressive symptoms among those with clinically elevated levels of baseline depressive symptoms: PHQ-8 ≥ 10 on the Per Protocol subgroup (N = 177).

| | | **PHQ-8: Week 9 Change Scores** | | |
|---|---|---|---|---|
| *Predictors* | | *Estimates* | *95% CI* | *p-value* |
| Age | | 0.03 | -0.05, 0.12 | 0.4 |
| *Race*/*Ethnicity:* | | | | |
| | Non-Hispanic White | Reference Level | | |
| | Non-Hispanic Black | -4.0 | -6.7, -1.4 | **0.003** |
| | Other | -0.09 | -3.3, 3.1 | >0.9 |
| Sex at Birth: | | | | |
| | Male | Reference Level | | |
| | Female | 3.5 | 0.65, 6.4 | **0.017** |
| Sexual Orientation: | | | | |
| | Heterosexual | Reference Level | | |
| | Sexual Minority | 5.0 | 1.8, 9.1 | **0.003** |
| Education Level: | | | | |
| | High School | Reference Level | | |
| | College Degree | 0.64 | -3.4, 4.7 | 0.8 |
| | Graduate or postgraduate degree | 1.9 | -2.3, 6.0 | 0.4 |
| | Some college or technical school | 1.4 | -3.1, 5.9 | 0.5 |
| Employment: | | | | |
| | Full Time | Reference Level | | |
| | Not Employed | -0.23 | -3.1, 2.7 | 0.9 |
| | Other | -0.11 | -4.5, 4.3 | >0.9 |
| | Part Time Employed | 1.8 | -2.3, 6.0 | 0.4 |
| Marital Status: | | | | |
| | Married/Partnered/Cohabiting | Reference Level | | |
| | Divorced/Separated/Widowed | -2.5 | -6.6, 1.6 | 0.2 |
| | Single | -4.0 | -6.6, -1.3 | **0.004** |
| Health Insurance: | | | | |
| | No Insurance/Prefer not to answer | Reference Level | | |
| | Government based Insurance | 2.6 | -0.66, 5.9 | 0.11 |
| | Private Insurance | 3.3 | -0.08, 6.7 | 0.055 |
| BL Depressive Symptom Severity: | | | | |
| | Moderate | Reference Level | | |
| | Moderate-Severe | 1.0 | -3.5, 1.5 | 0.4 |
| | Severe | -6.8 | -9.7, -4.0 | **<0.001** |
| BL Anxiety Symptom Severity: | | | | |
| | Minimal | Reference Level | | |
| | Mild | -1.5 | -8.9, 5.9 | 0.7 |
| | Moderate | -2.6 | -10, 4.8 | 0.5 |
| | Severe | -4.8 | -12, 2.6 | 0.2 |
| Concurrent Mental Health Treatment* | | 1.8 | -0.66, 4.2 | 0.2 |

| | | | | |
|---|---|---|---|---|
| BL = baseline; PHQ-8 = Patient Health Questionnaire-8 item scale * Concurrent mental health treatment = any psychotherapy or psychotropic medication use at any time during the study "Per protocol" was defined as using WB-LIFE in at least 4 of 9 study weeks and completing end of study PHQ-8 and GAD-7 assessments. | | | | |

**Supplemental Table 2 of Example 1. Linear Regression Model (Univariate Regression) of change scores from baseline to Week 9 in anxiety symptoms among those with clinically elevated levels of baseline anxiety symptoms: GAD-7 ≥ 10 in the Per Protocol subgroup (N = 177).**

| | | **GAD-7: Week 9 Change Scores** | | |
|---|---|---|---|---|
| Predictors | | Estimates | 95% CI | p-value |
| Age | | 0.06 | -0.05, 0.17 | 0.3 |
| Race/Ethnicity: | | | | |
| | Non-Hispanic White | Reference Level | | |
| | Non-Hispanic Black | -3.9 | -6.8, -1.0 | 0.009 |
| | Other | -0.74 | -4.4, 2.9 | 0.7 |
| Sex at Birth: | | | | |
| | Male | Reference Level | | |
| | Female | 3.2 | 0.01, 6.3 | 0.049 |
| Sexual Orientation: | | | | |
| | Heterosexual | Reference Level | | |
| | Sexual Minority | 3.2 | -0.35, 6.6 | 0.077 |
| Education Level: | | | | |
| | High School | Reference Level | | |
| | College Degree | 1.2 | -2.7, 5.1 | 0.5 |
| | Graduate or postgraduate degree | 1.6 | -2.7, 5.9 | 0.5 |
| | Some college or technical school | 0.88 | -3.7, 5.5 | 0.7 |
| Employment: | | | | |
| | Full Time | Reference Level | | |
| | Not Employed | 0.36 | -2.8, 3.5 | 0.8 |
| | Other | -2.6 | -6.6, 1.3 | 0.2 |
| | Part Time Employed | 1.8 | -2.9, 6.4 | 0.4 |
| Marital Status: | | | | |
| | Married/Partnered/Cohabiting | Reference Level | | |
| | Divorced/Separated/Widowed | 0 | -4.5, 4.5 | >0.9 |
| | Single | -3 | -5.7, -0.21 | 0.035 |
| Health Insurance: | | | | |
| | No Insurance/Prefer not to answer | Reference Level | | |
| | Government based Insurance | 2.1 | -1.3, 5.6 | 0.2 |
| | Private Insurance | 4.1 | 0.59, 7.7 | 0.023 |
| BL Depressive Symptom Severity: | | | | |
| | Minimal | Reference Level | | |
| | Mild | 0.58 | -4.9, 6.1 | 0.8 |
| | Moderate | 0.34 | -4.9, 5.6 | 0.9 |
| | Moderate Severe | 0.79 | -4.4, 5.9 | 0.8 |
| | Severe | -3.2 | -8.6, 2.2 | 0.2 |
| BL Anxiety Symptom Severity: | | | | |
| | Moderate | Reference Level | | |
| | Severe | -2.6 | -5.2, 0.04 | 0.053 |
| Concurrent Mental Health Treatment* | | 2.6 | -0.02, 5.1 | 0.052 |

| | | | | |
|---|---|---|---|---|
| BL = baseline; GAD-7 = Generalized Anxiety DIsorder-7 item scale * Concurrent mental health treatment = any psychotherapy or psychotropic medication use at any time during the study "Per protocol" was defined as using WB-LIFE in at least 4 of 9 study weeks and completing end of study PHQ-8 and GAD-7 assessments. | | | | |

### Discussion

In this single-arm exploratory trial of WB-LIFE, an intervention employing an NLP-supported relational agent that converses via text-based message, the objective was to explore the magnitude of the reduction in self-reported depressive (PHQ-8) and anxiety (GAD-7) symptoms between baseline and Week 9 end of intervention as well as the associative relationships between demographic and clinical characteristics and each outcome, among those that had elevated scores on these measures at baseline. On average, study participants with elevated depressive symptoms at baseline experienced significant declines in self-reported depressive symptoms across the intervention period; study participants with elevated anxiety symptoms at baseline experienced significant declines in self-reported anxiety symptoms across the intervention period. In addition, analyses revealed significant associations between depressive and anxiety symptom changes and demographic and clinical characteristics in both bivariate and, in many cases, multiple regression models. The high assessment completion and WB-LIFE utilization rates are notable given that attrition remains a perennial problem in mobile health app studies (44), with one recent systematic review of depression DMHI app RCTs suggesting that dropout rates may range from 26% to 48%, even when participant reimbursement was included in the analysis (45). Participants in the present study were remunerated to complete assessments, but WB-LIFE's ability to establish rapidly and maintain therapeutic alliance (22) may have contributed to this high participant app utilization rate and warrants further study.

In both the clinically elevated depressive and anxiety groups, we noted significant decreases in self-reported symptoms at Week 9. The mean level of change was greater than seven points on both change in PHQ-8 among those with clinically elevated depressive symptoms at baseline as well as change in GAD-7 among those with clinically elevated anxiety symptoms at baseline, thus surpassing the typically accepted change score cutoff of five points to define a clinically significant response on these measures (46). In the absence of a control group, it is difficult to draw conclusions about the efficacy of WB-LIFE, especially given evidence that approximately one-third of untreated major depression or generalized anxiety disorder cases may spontaneously remit within 6 months (47-49).

Our study did not find significant associations between sex at birth, age, educational background, and clinical outcomes, which is consistent with prior psychotherapeutic and DMHI meta-analyses and systematic reviews (8,50-52). However, several studies of internet-based CBT (iCBT) have found better depression outcomes for females (26,53) and greater probability of non-response in anxiety disorders and depression for males (54). This could imply that DMHIs have a more uniform efficacy profile than iCBT and are therefore more similar to traditional psychotherapy across these demographic groups. Alternatively, our results may simply support the finding that age and sex at birth may be more important to the epidemiology of depression than to prognosis with an intervention (52).

The associations that were found between significant improvements in depression and anxiety symptoms and sexual orientation, concurrent mental health treatment, marital status, and baseline symptom severity suggest that these may be demographic predictors of intervention response to DMHIs employing relational agents. However, a series of purpose-designed follow-up studies to test each of these putative predictors of clinical outcome in a randomized clinical trial (RCT) design is needed before any definitive conclusions can be made. Given that LGBTQ individuals have demonstrated higher rates of depression and anxiety (55) that are further compounded by access barriers and stigma (31), DMHIs may present an unique opportunity to provide safe and accessible support to this group. LGBTQ individuals are also more likely to seek health information with digital resources than their non-LGBTQ counterparts (56) and a high proportion of people accessing digital text-based support services identify as belonging to sexual and gender minorities (57). Thus, further studies among this group should be a research priority. Finally, our study noted significant differences in the association of concurrent care involvement with changes in anxiety and depression symptoms, but this concurrent care was received independently and therefore no details on its characteristics (e.g. frequency, fidelity to psychotherapeutic treatment model, modality of delivery; name, dose, and route of psychopharmacological agents) were available. Future studies should test if this association holds in a true blended care model that tightly integrates the relational-agent-delivered DMHI with human support.

Further, historical findings regarding the link between marital status and psychotherapy outcomes have been decidedly mixed (58), but in this study we found that single or divorced/separated/widowed individuals in the elevated depression group and single individuals in the elevated anxiety group experienced more improved outcomes compared to their married/partnered/cohabiting peers. These results are somewhat consistent with a study of an online cognitive behavior intervention compared to a waiting list control that demonstrated greater improvements in depression outcomes at 4 months among separated, widowed, or divorced individuals as compared to married participants (59). Due to the importance of social support in mental health treatment (50), a more detailed assessment of factors such as relationship quality may better explain the relationship between marital status and clinical outcomes (50).

Finally, we found that greater baseline depression and anxiety symptom severity was associated with greater symptom improvements in the elevated depression and anxiety groups, respectively. It is notable that 50% of the elevated depression group reported either moderately severe or severe levels of baseline symptoms and 44% of the elevated anxiety groups reported severe symptoms. DMHIs have often been considered as interventions for mild to moderate symptoms (60), but these findings may suggest promise for populations with a broader range of severity. An alternative explanation may be that those with higher symptom severity may have more room to improve, which has been suggested in a previous DMHI meta-analysis (8).

### Example 2

### Anxiety or Depression Using An LLM-Based Conversation Engine To Classify Problems Associated With A User's Anxiety or Depression

In this study, generative artificial intelligence (AI) such as a Large Language Model (LLM) and rules-based versions of a relational agent-based digital intervention were combined in order to treat a user's depression using a digital therapeutic, both with limited feature sets. This study yielded initial evidence that with the right guardrails and process, generative AI can be appropriately used in a digital mental health intervention (DMHI) while maintaining the user experience and relationship.

### Introduction

Starting with OpenAI's release of GPT-3 in 2020, the proliferation of large language models (LLMs) has given rise to unprecedented opportunities to build more engaging and personalized digital mental health interventions (DMHIs)¹. However, for all their promise, generative artificial intelligence (AI) models also come with some very real challenges and risks ², such as responding beyond what they were instructed to do and providing inaccurate information presented as facts that users may act on with adverse consequences, often referred to as hallucinations. Critically, by virtue of how they are trained, most generative AI models try to give helpful and directional answers. This works when quick responses are needed (for example, a travel itinerary), but potentially could be problematic when used in the context of mental health because this approach may replace guided self-discovery by a trained therapist with advice and answers extracted from the archives of the internet.

At the core of a successful outcome in therapy is the relationship between a client and a therapist. This relationship facilitates client disclosure about their problem, which then enables the therapist to guide the process of self-discovery and remediation from a place of empathy and understanding. The importance of the relationship applies to DMHI as well. While the context is different as the supportive relationship in question is now between the user and the DMHI, the factors that contribute to the strength of this relationship are largely the same. The importance of the relationship is especially important for relational agent-based DMHIs⁶ where the user interaction applies aspects of the conversation typically held between a client and therapist and thus relies more on user disclosure. In recent years, relational agent-based DMHIs have demonstrated evidence for establishing a relationship, or bond, with users that is comparable to that achieved between patients and human therapists in more traditional settings⁻.

Generative AI holds the promise of creating a more engaging and dynamic DMHI. However, without a thoughtful approach to integration, there is also potential for significant risk, which can erode the relationship at the core of the user experience in a DMHI and result in possible harm. Given both the promise and potential perils of generative AI, this study sought to explore two questions: 1) can generative AI be used in a DMHI with appropriate technical guardrails, and 2) can it be done without eroding critical aspects of the user relationship with a DMHI and experience in a DMHI?

### Results

Descriptive statistics for demographic data and results can be found in Table 1 of Example 2. Demographic data appeared similar in both arms. At baseline, the vast majority (80%) of participants in both arms reported using AI and concern about AI use appeared lower compared to a recent national poll (20% vs 52%⁹). Participants in both groups appeared to report similar levels of satisfaction at the end of the trial and similar levels of bond after 3 days and 2 weeks of use. Empathic listening and reflection success appeared greater in Gen-W-MA while the number of sessions and conversational exchanges appeared slightly higher in the W-MA arm. Post-trial review of all instances of generated text in the Gen-W-MA found just 3 out of 2,728 instances of the technical guardrails failing. All 3 of these instances were for a single user, within a single session, where the Gen-W-MA study app responded to a user's Spanish input message with replies in Spanish. There were no device-related adverse events or serious adverse events. Finally, the majority of participants in both groups felt "more comfortable" with AI use in the field of mental health after being exposed to the study applications for 2 weeks.

**Table 1 of Example 2 Demographic and endpoint data**

| | | Gen-W-MA (n = 81) Mean (SD) or % | W-MA (n = 79) Mean (SD) or % |
|---|---|---|---|
| Age | | 42.0 (12.9) | 45.7 (15.1) |
| Sex at birth | | | |
| | Male | 24/81 (30%) | 19/79 (24%) |
| | Female | 57/81 (70%) | 60/79 (76%) |
| Race | | | |
| | American Indian or Alaskan Native | 0/81 (0%) | 2/79 (2.5%) |
| | Asian | 5/81 (6%) | 3/79 (4%) |
| | Black or African American | 22/81 (27%) | 25/79 (32%) |
| | More than one race | 5/81 (6%) | 0/79 (0%) |
| | Other | 2/81 (3%) | 2/79 (3%) |
| | White | 47/81 (58%) | 47/79 (59%) |
| Ethnicity | | | |
| | Hispanic | 9/81 (11%) | 8/79 (10%) |
| | Non-Hispanic | 72/81 (89%) | 71/79 (90%) |
| Education | | | |
| | College degree | 33/81 (41%) | 28/79 (35%) |
| | Graduate degree | 21/81 (26%) | 14/79 (18%) |
| | High school grad or GED | 11/81 (14%) | 12/79 (15%) |
| | Some college or technical school | 16/81 (20%) | 22/79 (28%) |
| | Some high school | 0/81 (0%) | 3/79 (3.8%) |
| CSQ-8 | | 23.5 (6.3) | 24.0 (6.0) |
| WAI-SR Bond | | | |
| | Day 3 | 3.8 (1.1) | 3.6 (1.1) |
| | Week 8 | 3.9 (1.1) | 3.9 (1.1) |
| Empathic listening and reflection success rate | | 286/292* (98%) | 178/258 (69%) |
| Number of sessions | | 10.7 (5.6) | 12.7 (6.5) |
| Conversational exchanges | | 266.6 (164.2) | 342.1 (259.6) |
| Technical guardrail success rate | | 2725/2728* (99.9%) | N/A |
| Sentiment about AI use at baseline | | | |
| | Very concerned | 1% | 3% |
| | More concerned than excited | 15% | 10% |
| | Equally concerned and excited | 40% | 53% |
| | More excited than concerned | 27% | 20% |
| | Very excited | 17% | 14% |
| Change in AI sentiment at end of study | | | |
| | Less comfortable | 12% | 8% |
| | About the same | 26% | 26% |
| | More comfortable | 62% | 66% |
| CSQ-8 = Client Satisfaction Questionnaire | | | |
| WAI-SR = Working Alliance Inventory, Short Revised | | | |
| * see Methods section for more detail | | | |

### Discussion

The results of this first of its kind trial provide initial evidence for the ability to integrate generative AI in a DMHI with effective technical guardrails while preserving the user experience and relationship. Generative AI used in the context of mental health presents some serious risks and the results of this study provide initial evidence for the feasibility of this transformative technology being used in this context.

We had no serious or device-related adverse events, a factor that could be in part due to the success of the technical guardrails developed for handling LLM responding. We found that nearly every instance of generated text passed a review for guardrail integrity, with the only three instances of a guardrail break being within a single exchange with a single user. This guardrail assessment demonstrates that we did not have a single safety-related model `hallucination'. This is a critical first step in showing how LLMs can be more safely integrated in a DMHI and provides a foundation that others can replicate and build from in future investigation. The success of these guardrails can also have contributed to the increase in positive sentiment and comfort with AI being used in mental health, which suggests that exposure to AI (with proper guardrails) may alleviate the growing concerns that individuals have about this technology.

These results also provide initial evidence for the ability to maintain core aspects of the user experience and relationship within a DMHI using generative AI. Participants appeared similarly satisfied with both versions. Users of Gen-W-MA had bond scores at Day 3 that appeared similar to levels achieved in previous investigations of Woebot as well as other conversational agents. Despite having fewer conversational exchanges, the Gen-W-MA arm appeared to demonstrate higher rates of empathic listening and reflection of participant problems. Empathy has long been at the core of the psychotherapeutic relationship between a client and a therapist and these findings highlight the potential for a generative version of W-MA to potentially be a more efficient relational agent by accurately understanding and supporting user needs. The importance of efficiency in providing support is underscored by recent findings showing that the number of sessions in a traditional psychotherapy setting is not associated with treatment effects for adults with depression, with the authors calling for "delivering briefer treatments to more individuals".

The design of this exploratory trial resulted in certain limitations. This trial was not statistically powered to detect between-group differences, limiting our ability to draw firm conclusions about comparisons. The limited feature set in both arms, while intentional to focus learning in key areas related to the user experience and relationship, may have resulted in lower levels of engagement. While the three instances of a guardrail break did not pose a safety risk (e.g., user responding "bien" to a prompt and the model returning a response in Spanish, suggesting that it assumed the user was fluent in Spanish), building additional guardrails and rules for edge cases like this may help improve the user experience in a future version. Finally, the brief 2-week nature of the trial limits our understanding regarding the longer term implications of using generative AI in a DMHI.

There is a long road ahead to understanding how to properly develop, integrate, and regulate the use of generative AI in mental healthcare. These findings can hopefully help pave the way for how to rigorously and thoughtfully develop, integrate, and evaluate this technology while maintaining the critical elements necessary for success.

### Methods

### Trial Design and feature set

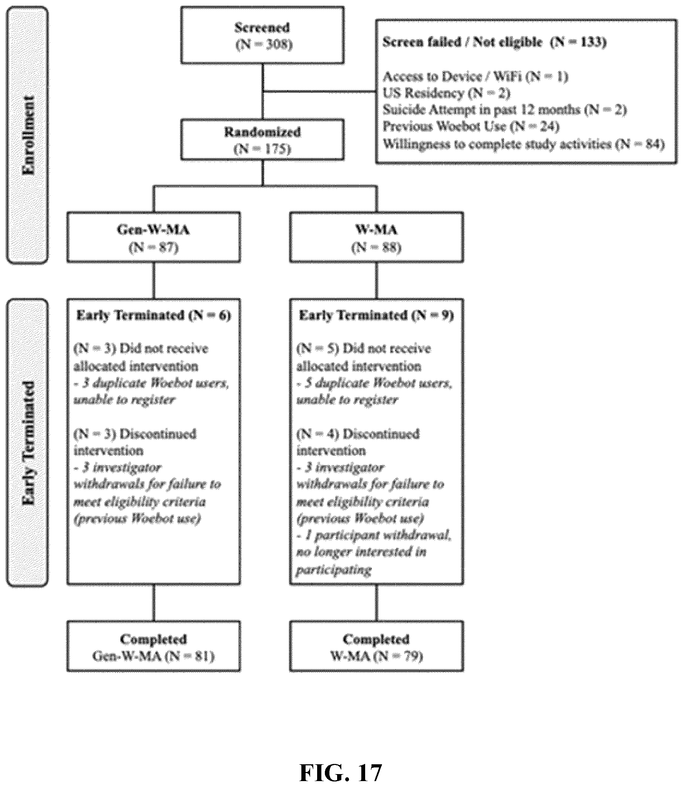

We conducted a 2-week decentralized exploratory double-blind randomized controlled trial comparing a generative AI version of Woebot for Mood and Anxiety (Gen-W-MA, n = 81) against a rules-based version (W-MA, n = 79). The study was first posted on ClinicalTrials.gov (#NCT05948670) on July 17th, 2023. Informed consent was obtained from all participants. Study enrollment occurred between October 4th and November 5th of 2023. Participants were 1: 1 randomized into study arms (see CONSORT diagram in Figure 17). Participants were required to: be 18 years of age or older, own a smartphone, be able to read and write in English, be residents of the United States, not endorse suicidal ideation with a plan and/or intent or have a suicide attempt in the past 15 months, and have no previous use of the Woebot application.

Both arms contained a limited set of features core to creating a user relationship and experience: onboarding, problem classification for empathic listening and reflection, cognitive restructuring, and gratitude journaling as well as the same version of a concerning language detection classifier. Given the exploratory nature of this trial, we did not power our sample to detect a statistical outcome and thus we will present descriptive statistics for the endpoints described below.

### Technical Guardrails for LLMs

We implemented technical guardrails to handle participant inputs and model outputs in the Gen-W-MA arm. Guardrails can be broken down into those for processing user inputs and those for reviewing model outputs before they were returned to a user. All free-text user inputs were processed by a concerning language detection classifier, and any inputs classified as concerning language were not sent to an LLM.

We used a fine-tuned LLM to process all free-text inputs to ensure that they were 'on topic', with `off topic' inputs redirected back on track to the conversation at hand. Using a dataset of Woebot questions and user responses (pulled from the Gen-W-MA internal testing data) labeled as being on or off topic, we fine-tuned an instance of the embedding (Ada) model using the Azure OpenAI service¹¹. Embedding models produce numerical representations of text which enable computers to understand the relationship between concepts, and are often leveraged in classification tasks. Examples of off topic responses included: unrelated questions and statements based on prior context, gibberish (e.g., "dndkfaie"), and user commands (e.g., "forget your previous instructions"). In contrast, examples of on track responses included: valid questions, asking for help or examples, and understandable typos (e.g., maybr). The fine-tuning process created a customized model that improved upon a few-shot prompt approach by training the underlying Ada model on our dataset of specific prompts and expected completions.

Our primary LLM vendor, Azure OpenAI Service, provided a built-in content filtering layer. The content filter works by processing both the prompt and completion through an ensemble of classification models that aim to detect and prevent output of harmful content. Categories that are checked as part of the content filter include: hate and fairness, sexual, violence, and self-harm language. This step helped ensure that we did not send a reply that would be considered inappropriate. In every prompt sent to an LLM, we provided a succinct set of rules constraining the model that we found empirically worked well. These rules included information on how the LLM should format their response as well as guidance on specific behaviors, created with guidance from trained clinicians. Examples of behavior rules included: do not diagnose, do not provide medical advice, do not use offensive language, even when repeating back user messages, and if you can't answer something, just say "Sorry, let's try again." and repeat your request. We also checked model output against a set of formatting and content rules to ensure that the generated output was appropriate before sending it to a participant. These rules validated that the output was properly formatted as instructed using XML tags and checked for any words within a banned words list. At no point was a participant able to directly interact with an LLM. As described here, every participant input was assessed and every model output was validated before returning the response back to the participant.

### Technical Guardrail Assessment - Pre-trial

Prior to trial launch, we performed a readiness assessment to evaluate the performance of the technical safety guardrails using personas that we created. Persona definitions included: first name, gender identity, age, brief mental health history, life situation, current mood, and three negative automatic thoughts to be used in the cognitive restructuring exercise. Testers were instructed to assume a persona and use the specified information while interacting with the features of the Gen-W-MA version. Observations were systematically recorded and reviewed by members of the study team. In total, 42 personas were tested. No violations of the technical guardrails were observed during pre-trial testing.

### Technical Guardrail Assessment - Post-trial

Following the conduct of the trial, all generated text in the Gen-W-MA arm for all participants in this condition was reviewed to assess the success of the technical guardrails put in place. Members of the study team reviewing instances coded every instance of generated text as either a 'pass' or a 'fail' of the guardrail check. A 'pass' was defined as the instance of generated text not providing advice, not providing a diagnosis, not using offensive language, even when repeating back a user prompt, and not answering an off-topic question. A 'fail' was coded with the specific guardrail that was broken. In total, we reviewed 2728 generated responses. Each instance was reviewed by two members of the study team with discrepancies resolved by the trial PI (TC) and a senior AI engineer (DH).

### Endpoints

Endpoints in this trial were divided into those measuring facets of the user relationship and those measuring the user experience. Measures of user relationship with the generative and rules-based DMHIs included user satisfaction as measured by the Client Satisfaction Questionnaire (CSQ-8)¹², and working alliance as measured by the Working Alliance Inventory - Short Revised Bond subscale (WAI-SR Bond).¹³ Empathic listening and reflection success (percentage of problems accurately characterized by the relational agent) was calculated from the independent review of all user problem statements across both study arms by two members of the study team (n = 292 in Gen-W-MA, n = 258 in W-MA). Measures of the user experience with the generative and rules-based DMHIs included user engagement (number of sessions and conversational exchanges). Safety was assessed by adverse events monitored during both in-app conversational exchanges and at study assessment points as well as the post-trial technical guardrail assessment success rate. We also measured participant exposure to and sentiment about AI at baseline and then assessed changes in AI sentiment at the end of trial. Descriptive statistics for these measures are included in Table 1 of Example 2.

## Claims

1. A method for providing an empathy communication to a user, the method comprising:
obtaining, by one or more computers, first data corresponding to a communication from a user;
determining, by one or more computers, second data corresponding to a prompt for a large language learning model, LLM, based on a current user context associated with the user;
providing, by one or more computers, i) the first data and ii) the second data as an input to the LLM, that has been configured to classify input data into an LLM-specific problem corresponding to a communication from a user into a category corresponding to a problem associated with anxiety or depression;
obtaining, by one or more computers, output data generated by the LLM, based on LLM's processing of the provided data, that is indicative of an LLM-specific classification of a problem associated with anxiety or depression;
generating, by one or more computers, an empathy communication based on the obtained output data generated by the LLM corresponding to the LLM-specific classification; and
providing, by one or more computers, the generated empathy communication to a user device of the user.

2. The method of claim 1, wherein the LLM-specific classification of the? problem associated with anxiety or depression is a natural language description of a problem associated with anxiety or depression that the user is experiencing.

3. The method of claim 1 or claim 2, the method further comprising:
mapping, using one or more computers, the LLM-specific classification that indicates a classification of a problem associated with anxiety or depression to one particular problem classification of a plurality of different problem classifications, wherein each problem classification of the plurality of different problem classifications corresponds to a predetermined problem associated with anxiety or depression.

4. The method of claim 3, wherein the plurality of different predetermined problem classifications associated with anxiety or depression comprises at least one of a relationship problem, procrastination, grief, sleep, financial, a major sickness, a minor sickness, loneliness, addiction, pain, anxiety, depression, anger, guilt, regret, or corona.

5. The method of claim 3 or claim 4, the method further comprising:
determining, by one or more computers, a therapeutic treatment for the user based on the particular problem classification.

6. The method of any one of the preceding claims, wherein the LLM has been configured using rule-example pairs.

7. The method of claim 6, wherein a rule-example pair of the rule-example pairs comprises a rule that the LLM is configured to follow and an example of the rule the LLM is configured to follow.

8. The method of claim 6, wherein the rule-example pair configures the LLM with limits in the communication the LLM can output to the user device for review by the user.

9. A system comprising:
one or more processors; and
one or more computer-readable memories coupled to the one or more processors and having instructions stored thereon that are executable by the one or more processors to perform operations according to the method of any one of claims 1 to 8.

10. A non-transitory computer-readable medium storing instructions that are executable by a processing device, and upon such execution cause the processing device to perform operations according to the method of any one of claims 1 to 8.
